# EUROPEAN PATENT APPLICATION

(11) **EP 1 733 684 A1**
(43) Date of publication of application: **20.12.2006**
(21) Application number: 05728794.8
(22) Date of filing: 05.04.2005
(51) Int. Cl.: A61B 10/00, A61B 17/34, A61M 1/00

(54) **METHOD OF COLLECTING BONE MARROW AND MEDICAL DEVICE FOR USE THEREIN**

(30) Priority: 05.04.2004 JP 2004111626
(71) Applicant: NIPPON CABLE SYSTEM INC., Takarazuka-shi Hyogo 665-0845 (JP); Noishiki, Yasuharu, Yokohama-shi, Kanagawa 236-0005 (JP)
(72) Inventor: NOISHIKI, Yasuharu;, okohama-shi, Kanagawa 2360005 (JP); FUJITA, Y., Nippon Cable System Inc., Takarazuka-shi, Hyogo 66500845 (JP); AMETANI, A., Nippon Cable System Inc.,, Takarazuka-shi, Hyogo 6650845 (JP); SEKI, Y., Nippon Cable System Inc., Takarazuka-shi, Hyogo 6650845 (JP)
(74) Representative: Bailey, David Martin
(86) International application number: PCT/JP2005/006653
(87) International publication number: WO 2005/096952

(57) **Abstract**

Using a bended puncturing needle, a through hole from the greater trochanter of a long tubular bone to the bone marrow cavity is formed, the catheter 61a, 61b is passed into the through hole, a physiological salt solution is injected from one of the catheter 61b, and the physiological salt solution and the bone marrow is stirred by the stirring wire 68, the obtained mixed liquid is aspirated and harvested by another catheter 61 a, thereby a large amount of the bone marrow is harvested. A method enabling easy and a large amount harvesting of the bone marrow from long tubular bones such as femurs, in which a multi-lumen catheter can also be used as a catheter.

## Description

### Field of the Invention

This invention relates to a method for harvesting the bone marrow and its medical apparatus, and more particularly to a method for harvesting the bone marrow from the bone marrow cavity of the long tubular bone and puncturing needles, catheters, and a medical apparatus set suitable for the method.

### Background Arts

[Non-patent Literature 1] Thomas etal.: Techniqe for human marrow grafting. Blood, pp.507, 1970
[Non-patent Literature 2] Ikehara etal.: A new method for bone marrow cell harvesting. Stem Cells, 18:6.pp453, 2000
[Non-patent Literature 3] Japan Society of Computer Aided Surgery, 2001 Report pp101-102 Kohta Ohhashi, Others: Development of minimally invasive bone marrow harvesting device (The first Report: Study of femur bone marrow harvesting device: Puncturing needles for harvesting bone marrow and its harvesting device and method for harvesting bone marrow)
[Patent Publication 1] U.S. Patent No.5,368,046
[Patent Publication 2] Japanese Published Patent Application No.2003-116862
[Patent Publication 3] U.S. Patent No.6,315,737
[Patent Publication 4] Japanese Published Patent Application No.2001-309919
[Patent Publication 5] U.S. Patent No.6,579,264
[Patent Publication 6] U.S. Patent No.5,005,585

Harvesting the bone marrow is essential for the diagnosis of vascular disease particularly for malignant diseases such as leukemia. For this purpose, as reported in the Non-patent Literature 1, a method is employed in which a bone marrow puncturing needle having a sharp front edge is strongly stuck into a bone to break the cortical bone and the bone marrow is aspirated from the medullary cavity.

As for the development of the puncturing needle for easy aspiration of the bone marrow from the medullary cavity is described in the Patent Publication 1 etc. And of the many bone marrow puncturing needles known, a movable stopper preventing the puncturing needle from excess insertion when sticking with a strong forces is attached as a safety device to adjust the depth from the skin surface and the medullary cavity in bone puncturing at a constant distance, for example 5mm to 1cm apart from the front edge. In the Patent Publication 1 etc., respective ingenuity is exercised in the stopper mechanism.

Further, many disposal one-off products have been thought of. They have a sheathed core structure composed of an inner needle and an outer needle, in which the inner needle have a sharp blade at the front edge for puncturing. And the aspiration is employed by puncturing the bone by the inner needle, fixing the outer needle by penetrating the front edge of the outer needle through the cortical bone, extracting the inner needle, and connecting the injection syringe to the outer needle to aspirate the bone marrow liquid. Many of these one-off products are not equipped with the stopper mechanism. Such fundamental structures of simple technique are seen, for example, in Patent Publication 2 etc.

In any conventional art, since puncturing needles are suitable for bone puncturing which directly reaches the bone marrow cavity, generally flat bones such as sternum, iliac bone, sacrum bone etc. existing near the body surface are selected for harvesting the bone marrow. Since many of these bones exist in the region where body weight is not always loaded, they are not covered by thick muscles and only covered with thin skin etc. Therefore, it is easy to break a cortical bone of the flat bones by puncturing strongly to reach the medullary cavity. Further, since these flat bones near the body surface have small thickness and the bone marrow cavity is narrow, the quantity of the obtainable bone marrow is so small extent as 0.5ml, but it is enough for the diagnosis of diseases.

However, the bone marrow transplantation has recently become widely used as a new medical treatment in which the large volume of the bone marrow are necessary. For such requirements, conventional techniques are used in harvesting the bone marrow, but the amount obtained at a time is limited. Therefore, under present circumstance for harvesting the bone marrow of the amount of about 1000ml (including peripheral blood, if net bone marrow only, 10-20ml) from one bone marrow donor, more than 100 spots of puncturing, and on occasion more than 200 spots are carried out to the bones of whole body to collect little by little.

Accordingly, beyond question the burden of the doctor harvesting and the burden of the donor being harvested are increasing extremely. An improvement to harvest a large amount of the bone marrow at one time puncturing is required, but no technique has been developed.

There is the large bone marrow cavity in a large bone, which is easily speculated to have a large amount of the bone marrow. However, conventional bone marrow harvesting is carried out from small thin bones such as sternum, iliac bone etc. described above and not from femurs which are the largest bones of mammal or human, or further, from tibias. The reason is that perimeter of these long tubular bones is surrounded by large muscles making it difficult for the puncturing needle to approach the bone, and further that the surface of these bones is composed of thin but hard compact bone. There is the bone marrow right under the compact bone and the distance from the surface to the bone marrow is short. But since the compact bone is hard, it is difficult to stick the bone marrow puncturing needle so as to penetrate the compact bone for harvesting the bone marrow. It is natural that many of the tubular bones are often loaded by the whole body weight and are required to have mechanical strength resulting in the formation of such strong compact bones.

Regardless of such anatomy, since the tubular bones have a large amount of the bone marrow, Ikehara and others reported a method to harvest the bone marrow from the bones efficiently in the Non-patent Literature 2. According to this, a part of the tubular bone is punctured by a bone marrow puncturing needle and a separate part is punctured by another puncturing needle. And a circulating fluid is injected from one of the puncturing needle and aspirated from another to harvest the bone marrow by the circulating fluid. In addition, the similar method and the bone marrow harvesting set provided with rigid pipes and drills are disclosed in the Patent Publication 2. Further, in the Patent Publication 3, a technique having a drill structure in the front edge is disclosed, because a weaker force is required to screw in than to push in by force only. In the Patent Publication 4, a bone marrow harvesting set and a bone marrow needle provided with a pipe like cannula and a moving piece slidable within it is disclosed.

The method to circulate a fluid by providing puncturing needle in two points as described in the Patent Publication 2 is used in the veterinary area. In animals, because the body weight loaded to one long tubular bone is relatively light and further the compact bone of animals is thinner than human. The femur puncturing is relatively easy and possible. However, in this circulating fluid method, since two points puncturing of the hard compact bone is necessary, it has many disadvantages in the application to large mammal and human.

Whereas, even in a tubular bone, there exists a region not surrounded by muscle layers in the epiphysis. Furthermore, in such epiphysis, there is a region where the skin is thin. In this thin region, puncturing is relatively easy. More specifically, it is the greater trochanter in the femur. The cortical bone is not formed because whole body weight is not loaded to this region. In such epiphysis, there are thin cortical bones in place of compact bones. However, there is a thick layer of spongy bone under the cortical layer, which makes it difficult for the front edge of the puncturing needle to reach the medullary cavity, thereby the bone marrow aspiration were precluded.

Therefore, it went so far as to be said that no bone marrow exists in the tubular bone. However, in large bones, particularly in tubular bones, there is a large medullary cavity, and it is desirable for bone marrow donors who are required to be harvested a large amount of the bone marrow to make practical use of them, because it decreases number of puncturing region reducing the burden of the donors. But it has been impossible on the reason that there has been no medical apparatus to reach the medullary cavity existing in the deep region when puncturing from the greater trochanter region.

In the Non-patent Literature 3, a method to harvest the bone marrow from the epiphysis is further reported. The technique is to insert a system to pour a circulating fluid from the epiphysis, and its possibility of efficient harvesting is reported. According to this report, after puncturing a large hole in the epiphysis, a device composed of pipes of stainless steel-made multi-lumen is inserted through the hole. Specifically, BMC Harbester is inserted by Seldinger's catheter method and the device is inserted along the guide wire after reaching to the bone marrow cavity of a pig femur from epiphysis using an intramedullary reamer. The multi-lumen of the device is composed of a pipe for inserting the guide wire, a pipe for pouring the circulating fluid and a pipe to aspirate the bone marrow, and it aspirates while pouring the circulating fluid of a physiological salt solution. Therefore, the device has a hard and thick structure. For example the Seldinger's catheter method is described in the Patent Publication 5.

According to this report, it is described that a simulation experiment was performed using agar, assuming an acrylic pipe having 18mm inner diameter as a tubular bone, and that it was possible to harvest the agar in the region where the device passes through. However it was not possible to harvest the agar in the region apart from the device. Since the inside of bones is not smooth as the acrylic pipe, there is a limit in efficiently harvesting the bone marrow from the actual medullary cavity using this device. Further, such device tends to be thick and hard. Inserting such a thick device into the epiphysis necessitates perforating a large straight hole in the bone marrow cavity which exists in the depth of the epiphysis, and it causes heavy burden to be imposed on patients and donors, which may be concerned about departing from the purpose of reducing the burden.

The end of a tubular bone, specifically, the greater trochanter existing at the end of femur lies at the lumber region, is positioned at the nearest region to the skin in the end of the central side of the femur. In this point the compact bones are not formed, because it is not loaded with the body weight. So it is an easy point for puncturing. However, it needs a high technique to stick a hard and thick pipe into the bone marrow of the femur from this region passing through the thick spongy bone.

Further, on the occasion of inserting such a thick and hard pipe, the side damages will be large, if it is inserted into the region other than the bone marrow cavity. Therefore, it needs to see through by roentgen ray in puncturing so as to insert accurately. If it is stuck into the epiphysis fortunately, since the femur is not straight and have gradual curve, it is unreasonable to insert the rigid pipe to the depth of the medullary cavity. Furthermore, it needs the general anesthesia to perform such treatment, the burden of patients and donors further increases.

Accordingly, as far as the above described rigid pipe having stainless- made multi-lumen is used, it is difficult to insert efficiently to the every corner of the tubular bone. Furthermore, it is the present condition that efficiently harvesting of the bone marrow in the tubular bone while reducing the burden cannot be expected.

In addition, since many of the tubular bones lie in the direction of the body axis, in order to insert the rigid pipe in the axis of the tubular bone, the tubular bone must be strongly inflected against the body axis. For example, in the case of harvesting from the femur, the femur must be inflected and further strongly adducted. Therefore, the repositioning of the body under the general anesthesia causes troubles related to the anesthesia, and causes troubles related to cleanness. Further, there is an arthrosis in the end of the tubular bone in many cases. So there is a danger of occurrence of arthrosis disabilities, because in the insertion of such a thick pipe straight, the puncturing happens to reach the arthrosis cavity.

In the case of femur, the central side epiphysis is blanched out roughly into two, one of which is the caput forming articulatio coxae and another is the greater trochanter region. Since in this greater trochanter region there is no arthrosis. So, the puncturing of this region does not affect the arthrosis. However, even when a catheter is introduced by puncturing the greater trochanter with the puncturing needle, it is extremely difficult to stick the puncturing needle straight to the diaphysis. It is realistically difficult to puncture straight from the greater trochanter to the tubular bone axis, even when the femur is in parallel with the body axis; the shortest point from the skin is selected as a puncturing point; the femur is inclined by bending the leg inside; the trochanter region is made close to the skin as far as possible; and the center axis of the femur is staggered from the long axis of the body. Actually, even when this operation of puncturing is carried out while seeing through with roentgen rays, it is difficult to puncture straight to the tubular bone axis three dimensionally and securely. And it is extremely difficult only by the rigid puncturing needle to make reach the bone marrow cavity from the greater trochanter.

### Disclosure of invention

Depending on conventional arts, it is impossible to harvest the bone marrow in long tubular bones by direct puncturing. It is also impossible to sufficiently harvest even if two holes are perforated and the circulating fluid is been run through. Further, since the distance to the medullary cavity is long, puncturing from the epiphysis is impossible. To solve this problem, it has been conceived to insert a hard and thick tube from the epiphysis, but there have been problems in this method that the burden to patients is heavy and that an amount of bone marrow to be harvested is limited. This invention is directed to provide a bone marrow harvesting method and its medical apparatus used for the method, which enables efficient, plenty, and easy harvesting of the bone marrow in long tubular bones by small puncturing imposing light burden to patients or mammals to be treated.

The bone marrow harvesting method of this invention(claim 1), comprises a steps of forming a hole in a cortical bone, inserting a flexible catheter into a bone marrow cavity through the hole, and aspirating the bone marrow through the catheter. In this method, it is preferable to further comprise steps of injecting a dilute solution to the bone marrow cavity through the catheter, stirring the bone marrow and dilute solution, and aspirating the bone marrow as a mixed liquid (claim2). Further, it is preferable to comprise steps of passing a puncturing needle through from a epiphysis of a long tubular bone to a spongy bone or bone marrow cavity, inserting a guide wire into the bone marrow cavity through from a cavity of the puncturing needle, extracting the puncturing needle leaving the guide wire, inserting the flexible catheter with guidance of the guide wire, and aspirating the bone marrow through the catheter (claim 3). It is preferable to comprise the step of fixing or contacting a punctured hole securing member having a cavity to a cortical bone, before inserting the puncturing needle into the spongy bone or bone marrow cavity through the cavity of the punctured hole securing member (claim 4). Further, inserting an exploring tube, confirming that the exploring tube has reach the bone marrow cavity by aspirating the exploring tube, before inserting the flexible catheter after extracting the puncturing needle is preferable (claim 5). Moreover, inserting a needle like device having a cavity and bended front edge with a guidance of the guide wire, and further inserting the guide wire deeply into the bone marrow cavity using the needle like device may be further comprised (claim 6).

The first embodiment of the medical apparatus for harvesting the bone marrow (claim 7) is preferable to be a combined set of medical apparatus for harvesting the bone marrow comprising a a puncturing needle composed of an outer needle having a cavity provided with rigidity and an inner needle to be inserted into the outer needle; and a tube like flexible catheter inserted into the outer needle or a hole of a bone formed by the outer needle.

The second embodiment of the medical apparatus for harvesting the bone marrow (claim8) is preferable to be a combined set of medical apparatus for harvesting the bone marrow comprising a puncturing needle composed of an outer needle having a cavity provided with rigidity and an inner needle to be inserted into the outer needle; a guide wire which can be inserted into the outer needle of the puncturing needle; and a needle like device having a cavity for inserting the guide wire, having a bended part with the cavity, and capable of being inserted into a hole formed by the puncturing needle. Further, in these medical apparatus, it is preferable to be provided with a fixing means to the cortical bone and the punctured hole securing member having a cavity (claim 9).

The first embodiment of puncturing needle for harvesting the bone marrow (claim 10) is preferable to be provided with a cavity provided axially for inserting a guide wire, wherein the puncturing needle may be provided with a bended or without a bended part. In such puncturing needle, the front edge is formed into multi-pyramid cone and the cavity is opened at the vicinity of the front edge (claim 11). Further, the cavity is preferable to be opened at the inside of the bend of the puncturing needle (claim 12).

The second embodiment of the puncturing needle for harvesting the bone marrow (claim 13) is preferable to comprise an outer needle having a cavity provided axially for inserting a guide wire with the cavity opened at a front edge where the outer needle may be provided with a bended or without a bended part; a flexible inner needle accommodated rotatably and extractably in the cavity of the outer needle, wherein a front edge of the inner needle has a cone shape, and a base of the inner needle is protruded from the outer needle so as to be operatable when the inner needle is accommodated in the outer needle presenting a sheathed core structure as a whole. In such puncturing needle, it is preferable to comprise an interlock mechanism provided in a part of the outer needle, which allows a rotation or reciprocation of the inner needle and at least restrains an axial movement toward a back end (claim 14).

A catheter for harvesting bone marrow of this invention (claim 15) is used together with any of the needle described in claim 10 to 14, wherein an inner hole for a guide wire to be inserted and a flexibility to advance the periphery of the guide wire in bended condition are provided. In such catheter, coupling part to inter connect an aspirating means to be connected to the inner hole, a liquid injecting part, and switching means at least at a par of the near side are preferable to be provided leastwise at a part of the near side (claim 16).

The second embodiment of catheter for harvesting the bone marrow (claim 17) is preferable to comprise a lumen for injecting a dilute solution, and a lumen for aspirating a diluted bone marrow. The above catheter is preferable to be provided with a stirring part in the vicinity of the front edge (claim 18). The above catheter has preferably at least one lateral hole for aspirating the bone marrow in the vicinity of the front edge (claim 19).

The first embodiment of the guide wire for harvesting the bone marrow (claim 20) is the guide wire to be inserted into the catheter described in any of the above catheter, of which the one end has flexibility and another end has hardness or rigidity.

The second embodiment of the guide wire for harvesting the bone marrow (claim 21) is the guide wire to be inserted into the catheter described in any of the above catheter, of which the rigidity is substantially the same along the whole length.

The third embodiment of the guide wire for harvesting the bone marrow (claim 22) is the guide wire to be inserted into the catheter described in any of the above catheter, of which the guide wire is provided with a stirring part in the vicinity of the front edge.

The third embodiment of the medical apparatus for harvesting the bone marrow (claim 23) is preferable to be a combined set of any of the above puncturing needle, the guide wire to be inserted into the puncturing needle, and the bendable catheter being passed through the periphery of the guide wire. In such medical apparatus, the bendable front edge slidably attached to the periphery of the guide wire is preferably further provided with an exploring tube having hardness (claim 24) . These medical apparatus is preferable to be provided with a fixing means to the cortical bone, and the punctured hole securing member having a cavity.

### Effect of the Invention

In the bone marrow harvesting method of this invention, a hole is formed in a cortical bone and a flexible catheter is passed through the hole to aspirate the bone marrow through the catheter. Therefore, the aspiration and the harvesting can be done while moving the front edge of the catheter in the bone marrow cavity allowing a large amount of the bone marrow to be harvested. In this method, different devices may be used as the aspiration device and the puncturing device. For example, a catheter having a large inner aspiration diameter with the enlarged aspiration hole of the bone marrow under the same diameter hole of the bone may be used as the aspiration device. Thereby, the bone marrow is efficiently aspirated imposing lighter burdens to patients (claim 1).
In such method of harvesting the bone marrow, when a dilute solution is injected to the bone marrow cavity through the catheter and the bone marrow liquid and the dilute solution is stirred to aspirate the bone marrow at the time of harvesting the cone marrow (claim 2), the viscosity of the bone marrow is decreased by the dilute solution facilitating easy aspiration and the harvesting of a larger amount of the bone marrow.

when a puncturing needle is passed or stuck through from the epiphysis of a long tubular bone to a spongy bone or bone marrow cavity; a guide wire is inserted or passed through from the cavity of the puncturing needle to the bone marrow cavity; the puncturing needle is taken out leaving the guide wire; the flexible catheter is inserted into the bone marrow cavity with the guide of the guide wire; and the bone marrow is harvested through the catheter, since the hole is formed by the puncturing needle; it is possible to form the hole exactly reaching the bone marrow cavity in relation to the epiphysis can be formed. Further, since the catheter is guided by the guide wire, the catheter is easily and securely inserted into the bone marrow cavity. Furthermore, the puncturing needle is inserted into the cavity of the punctured hole securing member so as to stuck through to the spongy bone or bone marrow cavity after a punctured hole securing member having a cavity is fixed or contacted to a cortical bone, it is possible to prevent the puncturing needle from jumping laterally due to missing one's aim etc.

An exploring tube is inserted and advanced, and confirmation of the reach of the tube to the bone marrow cavity by aspirating the exploring tube before inserting the flexible catheter after taking out the puncturing needle as above(claim 5), the puncturing of the puncturing needle is enough to reach as far as around the spongy bone, because the puncturing of the spongy bone can be employed by the guide wire in which the puncturing needle is passed through. Thereby the puncturing is easy. Further, since it is possible to confirm the reach of the needle to the bone marrow previously by aspirating the exploring needle, useless preparation for harvesting can be saved. Furthermore, when a dilute solution is injected to the bone marrow cavity through the catheter prior to the bone marrow harvesting and the bone marrow liquid and the dilute solution are stirred before the aspirating of the bone marrow, a large amount of the bone marrow can be efficiently harvested.

When a needle like device having a cavity bended at its front edge is inserted with the guide of the guide wire and the guide wire is further deeply inserted into the bone marrow cavity by the needle like device after the puncturing needle is taken out(claim 6), the direction of the guide wire can be changed by the needle like device. Therefore, the puncturing needle is enough to be straight or to be the one having small bending, thereby the first puncturing work becomes easy.

The medical apparatus set of this invention (claim 7) is the combination of a puncturing needle composed of an outer needle having a cavity provided with rigidity and an inner needle to be inserted into the outer needle, and a tube like flexible catheter. So, it is possible to form a hole in a cortical bone, to take out the inner needle, and to insert or pass the catheter through the outer needle or the hole formed by the outer needle so as to aspirate the bone marrow. Thereby a large amount of the bone marrow can be easily harvested.

The second embodiment of the medical apparatus of this invention (claim 8) is provided with the needle like device having bended part, so the direction of the guide wire can be changed. Therefore it is possible to insert the guide wire, which guides the catheter, deep into the depth of the medullary cavity.
In the above medical apparatus set, when a fixing means to the cortical bone and the punctured hole securing member having a cavity are provided, it is possible to puncture safely to bones preventing the puncturing needle to jump laterally due to missing one's aim (claim 9).

The puncturing needle of this invention (claim 10) has at least a bended part partially, so it is possible to stick the puncturing needle from the side apart from the arthrosis region in the epiphysis of the tubular bone and to perforate a bended hole to the epiphysis making the front edge reaching the bone marrow cavity. More specifically, since there is an arthrosis in the epiphysis and the long axis of the bone is the same as that of the body axis, it is difficult to puncture straight along the long axis from the end of the bone. But when the needle of this invention is used, the puncturing needle can be stuck from the side of the epiphysis, and then the front edge of the needle can be gradually changed toward the medullary cavity in the bone to direct the front edge of the needle to the bone axis. And further, the bended path for passing through the guide wire can be secured by the cavity of the puncturing needle. In addition, the front edge of the puncturing needle may be advanced until it reaches the medullary cavity, or it may be advanced in so far as to reach inside the spongy bone. So the guide wire can penetrate the spongy bone into the medullary cavity by pushing.

The bone marrow can be harvested followed by inserting the guide wire into the cavity, extracting the puncturing needle, using of the exploring tube, and inserting the catheter. Thus the employment of the needle of this invention allows to puncture from the side of the epiphysis, particularly from the greater trochanter just under the skin, donors or patients are not required to bend the femur widely and further to twist inward. Therefore, the burden to donors and patients is light and also to doctors. The burden to donor is lighter and insertion is easier when the outer diameter is made to be small. Bringing a bend to the puncturing needle allows easier puncturing from the greater trochanter to the medullary cavity. Further, thinning the outer diameter of the puncturing needle makes the hole diameter of epiphysis remaining after the puncturing small, which lighten the burden of donors and patients.

Additionally as described above, since it is possible to puncture from the side of the epiphysis apart from the arthrosis, it is possible to puncture the neck bone or the splint bone of the lower thigh or humerus of the upper limb to harvest the bone marrow.

In the above puncturing needle, when the front edge is formed into multi-pyramid and the cavity is opened laterally in the vicinity of the front edge of the puncturing needle (claim 11), the cortex of the bone surface can be easily broken. The cortical bone existing on the bone surface of epiphysis is thinner than the compact layer of the diaphysis. Nevertheless it is the end part of the bone, the cortex of bone is hard and difficult to puncture. In the puncturing needle in which the cavity is opened laterally in the vicinity of the front edge and its front edge is formed into multi-pyramid, because the needle can easily break the cortex and can enter inside by applying and rotating the front edge to the bone surface, because the front edge is sharp and formed into multi-pyramid. Further, depending on the relation between the condition of the applied force and the passage of the needle, the possibility of the bone chips to be pushed into the opening becomes small. Furthermore, as described above, since the change of direction inside the bone utilizing the bended shape is possible, needle may be stuck by applying straight to the bone surface which prevents the slipness and enhance easiness of the puncture. This method enables to puncture the hard cortical bone without applying excess force. It is not always necessary to provide such a safety device as stopper preventing excess insertion as used in conventional techniques.

In the puncturing needle in which the cavity is opened inside of the bend of the puncturing needle (claim 12), the opening is directed to the cavity, even in the condition that the curve of the puncturing needle is not enough and its front edge is not directed straight to the medullary cavity, when the guide wire is pushed out in the spongy bone,. Therefore, there is a merit that in this condition the guide wire can be pushed out to the medullary cavity.

The second embodiment of the puncturing needle of this invention (claim 13) composed of the outer needle using the puncturing needle of above, and the sheathed core in which the inner needle is accommodated. It is possible to break the epiphysis and spongy bone by the inner needle and secure the bended path by the outer needle. Because the inner needle has flexibility, it can be advanced in the outer needle while being rotated and depending upon the case being reciprocated back and forth. Therefore, it can be advanced without being applied strongly allowing more easily puncturing to the epiphysis. Further, in the case that the inner needle is made to be extractable from the outer needle, when the inner needle is taken out after puncturing into the epiphysis, same as described above, the guide wire can be inserted into the cavity. And then, the guide wire can be used as a guide for loading the exploring catheter, or the loading of the catheter for harvesting the bone marrow.

In the puncturing needle (claim 14) having an mechanism in which the inner needle is interlocked by rotation is provided in a part of the outer needle, inner needle can be rotated at the same position, so the rotating of the inner needle may be concurrently employed with pushing of the outer needle along the axis, thereby facilitates efficient puncturing and handling.

Since the catheter of this invention (claim 15) has an inner hole to pass through at least one guide wire inserted into the cavity of the puncturing needle and is provided with the flexibility with which it can advance while being guided along the periphery of the bended guide wire, after passing the guide wire through the puncturing needle into the epiphysis and then taking out the needle, it can be advanced by loading around the remaining guide wire. Additionally, the catheter is so made as to be the multi-lumen type catheter provided with the inner hole other than that to pass through the guide wire, it can harvest the bone marrow liquid at the same time while the dilute solution such as a physiological salt solution are injected, and thereby the efficient harvesting of the bone marrow is possible.

In such catheter, in the case that coupling part to interconnect an aspirating means, liquid injecting part, and switching means are provided leastwise at a part of the near side (claim 16), the diluted bone marrow liquid can be harvested by connecting the inner hole to pass the guide wire and liquid injecting means, injecting the dilute solution such as a physiological salt solution to the medullary cavity and connecting to the aspirating means. Thereby efficient harvesting is possible.

The second embodiment (claim 17) of the catheter for harvesting the bone marrow of this invention is provided with the lumen for injecting a dilute solution and the lumen for aspirating the diluted bone marrow, so the aspirating and harvesting may be possible while injecting the dilute solution. Further, when the dilute solution is injected first and the aspirating and harvesting is done after, there is no necessity of switching the injecting apparatus and the aspirating apparatus. Therefore the efficient harvesting is possible. Furthermore, since it has a lateral hole in the vicinity of the front edge of the lumen for harvesting the bone marrow, a large amount of the dilute solution containing the bone marrow can be aspirated.

Additionally, in the case that the catheter is provided with a stirring part in the vicinity of the front edge (claim 18), after the dilute solution is injected to the medullary cavity, the dilute solution is widely spread in the medullary cavity by rotating or reciprocating the catheter at the near side. Thereby a large amount of the bone marrow in the medullary cavity can be harvested. Further, in the case that the catheter has the lateral hole for aspirating the bone marrow in the vicinity of the front edge (claim 19), (wherein, in the case of multi-lumen type, it is in the vicinity of front edge of the lumen for aspirating the bone marrow.) the aspiration can be carried out from the lateral hole in addition to the opening in the front edge, thereby efficient harvesting is possible.

Since in the first embodiment of the guide wire for harvesting the bone marrow of this invention (claim 20), the one end has flexibility and the another end has hardness or rigidity, after the catheter is inserted into the epiphysis of the object of harvesting, the guide wire can penetrate or push the spongy bone etc. by inserting into the catheter from the end having rigidity. Then after the resistance decreases abruptly, it is possible to confirm securely the reach of the catheter to the medullary cavity by extracting the guide wire and inserting the guide wire from the end having flexibility into the catheter having flexibility. In other words, when the guide wire is deeply inserted, it contacts the other end of the bone marrow, and it is possible to confirm that the catheter is inserted into the medullary cavity from the feel and from the length inserted. In the unlikely event that it did not reach the bone marrow cavity and the front edge of the catheter got out of the bone, it will not hurt the tissues out of the bone, because the front edge is soft. On the other hand, the guide wire having substantially the same rigidity along the whole length is easy to manufacture, and has a merit of being non-breakable and high-safety.

The guide wire provided with a stirring part in the vicinity of the front edge (claim 22) can stir the bone marrow in the medullary cavity and the dilute solution by rotating or reciprocating the guide wire passed through the catheter at the near side. Thereby the harvesting of a large amount of the bone marrow in the medullary cavity is possible. Further, since the guide wire is inserted into the catheter, such operation of rotation or reciprocating is easy.

Since the third embodiment of the set of medical apparatus of this invention (claim 23) is provided with the above described puncturing needle, the guide wire, and the catheter, the above method for harvesting and aspirating of the bone marrow can be carried out taking the effect of these apparatus. In the case that the exploring tube is added to the third embodiment of the set of the medical apparatus (claim 24), the reach to the medullary cavity can be early detected. Further, in the case that in such medical apparatus a fixing means to the cortical bone and the punctured hole securing material having a cavity is provided (claim 25), the puncturing needle can be held making the harvesting and aspirating of the bone marrow easy.

### Brief Description of Drawings

[Figure 1] Figure 1a and Figure 1b are a front view and a side view showing respectively an embodiment of the puncturing needle of this invention.
[Figure 2] Figure 2a and Figure 2b are a close-up side view and a close-up cross section showing respectively the front edge of the puncturing needle of this invention.
[Figure 3] Figure 3 is a side view showing an embodiment of the usage of the puncturing needle of this invention.
[Figure 4] Figure 4 is a cross section showing the conditions in use of the puncturing needle of Figure 1.
[Figure 5] Figure 5a and Figure 5b are a front view and a side view showing the other embodiment of the puncturing needle of this invention.
[Figure 6] Figure 6a and Figure 6b are a close-up side view and a close-up cross section showing respectively the front edge of the puncturing needle of this invention.
[Figure 7] Figure 7a and Figure 7b are substantial side views showing respectively the other embodiment of the puncturing needle of this invention.
[Figure 8] Figure 8 is a substantial close-up side view showing an embodiment of the inner needle used for the puncturing needle.
[Figure 9] Figure 9 is a substantial perspective view showing further the other embodiment of the puncturing needle of this invention.
[Figure 10] Figure 10 is a substantial perspective view showing further the other embodiment of the puncturing needle of this invention.
[Figure 11] Figure 11 is a substantial cross section of the conditions in use of the outer needle related to this invention.
[Figure 12] Figure 12a is a substantial close-up perspective view showing further the other embodiment of the puncturing needle related to this invention, Figure 12b is a substantial perspective view of the outer needle used for the puncturing needle.
[Figure 13] Figure 13a is a substantial perspective view showing further the other embodiment of the puncturing needle of this invention, Figure 13b is a substantial perspective view of the inner needle of the puncturing needle, Figure 13c and Figure 13d are substantial perspective views of the outer needle of the puncturing needle of Figure 13a.
[Figure 14] Figure 14a to Figure 14d are respectively substantial perspective views showing further the other embodiment of the outer needle related to this invention.
[Figure 15] Figure 15 is a side view showing a partial process in an embodiment of the method for harvesting the bone marrow of this invention.
[Figure 16] This is a side view showing a partial process of the method for harvesting the bone marrow continued from Figure 15.
[Figure 17] This is a side view showing a partial process of the method for harvesting the bone marrow continued from Figure 16.
[Figure 18] This is a side view showing a partial process of the method for harvesting the bone marrow continued from Figure 17.
[Figure 19] Figure 19 is a side view showing the other embodiment of the partial process drawing for harvesting the bone marrow of this invention.
[Figure 20] Figure 20 is a side view showing further the other embodiment of the partial process drawing for harvesting the bone marrow of this invention.
[Figure 21] Figure 21 a-d are respectively the side views showing further the other embodiment of the guide wire of this invention.
[Figure 22] This is a side view showing further the other embodiment of the catheter used for harvesting the bone marrow of this invention.
[Figure 23] Figure 23a and Figure 23b are respectively a substantial perspective view and a substantial cross section of the catheter of Figure 22, and Figure 23c and Figure 23d are respectively a substantial perspective view and a substantial cross section showing further the other embodiment of the catheter related to this invention.
[Figure 24] Figure 24 is a side view showing further the other embodiment of the catheter used for harvesting the bone marrow of this invention, Figure 24b and Figure 24c are respectively a substantial perspective view and a substantial cross section of the catheter of this invention.
[Figure 25] Figure 25 is a partial process drawing showing the other embodiment of the method for harvesting the bone marrow of this invention.
[Figure 26] Figure 26 is a partial process drawing showing the other embodiment of the method for harvesting the bone marrow of this invention.
[Figure 27] Figure 27a is a side cross section showing an embodiment of the punctured hole securing material used for this invention, Figure 27b is a side view showing the bone marrow puncturing system provided with the punctured hole securing member, Figure 27c is a side view of the parts comprising the bone marrow puncturing system.
[Figure 28] Figure 28 is a process drawing showing the method for harvesting the bone marrow of this invention.
[Figure 29] Figure 29 is a side view showing an embodiment of the punctured hole securing needle system provided with the punctured hole securing member of Figure 27a, and Figure 29b is a side view of the parts comprising the punctured hole securing needle system.

### Best mode for carrying out the invention

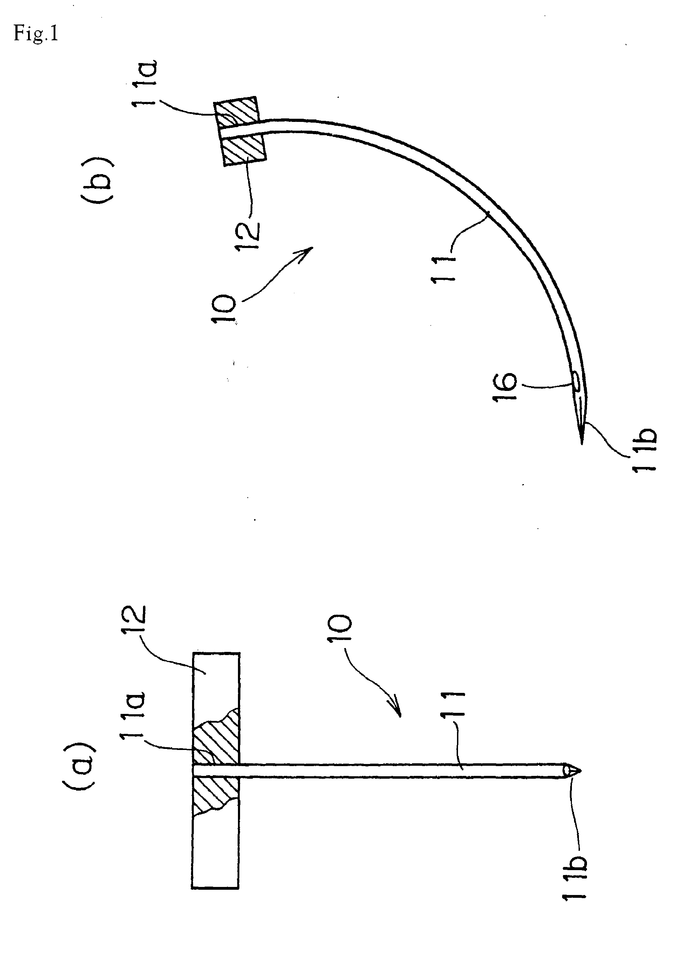

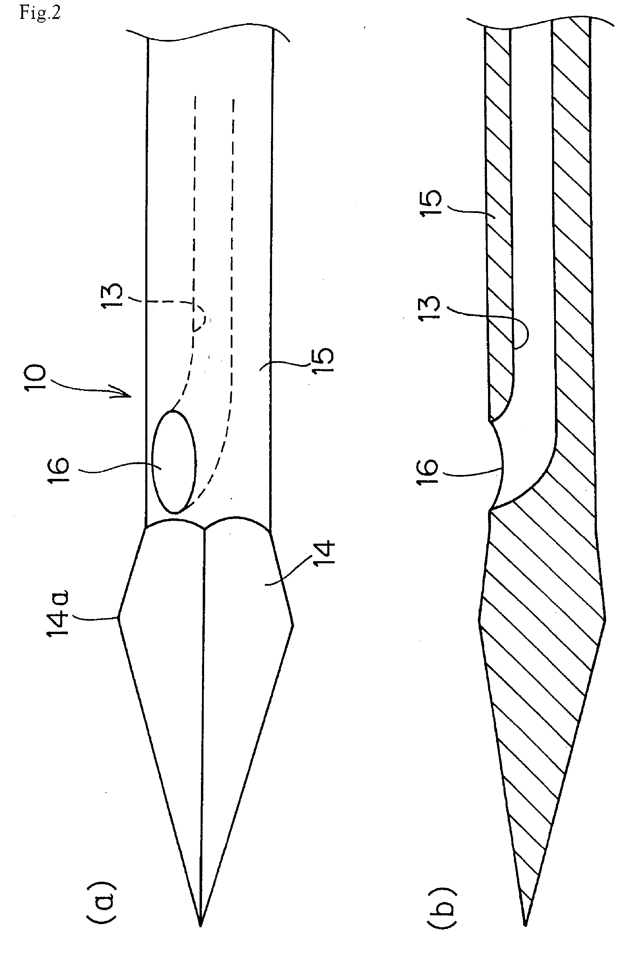

The embodiments of this invention are described referencing the figures. The puncturing needle 10 shown in Figure 1a and Figure 1b comprises a needle 11 bended like circular arc and a grip 12 attached to the base of the needle. The needle 11 is made of metal having high strength with no harmful effect to human body such as stainless steels, titanium alloy, precious metal alloy, and cobalt alloy. It is preferable to use metals which roentgen ray cannot transmit. The needle 11 presents tubular shape provided with a cavity (see reference numeral 13 of Figure 2b) from a base edge 11a to a front edge 11b. The needle 11 has such elasticity as bendable in some degree, but it is so rigid as to keep the bended shape fundamentally. The grip 12 presents a rod shape and its center is fixed to the periphery of the base edge 11 a of the needle 11. The material of the grip 12 is not particularly limited, but is made of metal or synthetic resin.

The front edge 11b of the needle 11 is, as shown in Figure 2a and Figure 2b, provided with a cutting part 14 having sharp cone shape or multi-pyramid shape. The cutting part 14 is preferable to be two-plane, three-plane, or four-plane. The ridgeline of the cutting part 14 is made to be a sharp cutter blade, and the outer diameter of the thick part 14a is larger than that of the other part 15 of the needle 11. The other part 15 is extended with practically the same diameter. The cavity 13 is provided concentrically with the diameter of the needle 11, and extending along the center of the needle to open directly along the axial in the base edge 11a. On the other hand, in the front edge 11b side, it is bended gradually before the cutting part 14, and opens in the vicinity of the edge part of the same diameter part 15. The opening part 16 is bended inward of the curvature and its direction is inclined toward the front side against the centerline of the needle 11. The cutting part 14 is manufactured separately to the other part 15, and attached to the other part by blazing etc., thereby allowing the use of high rigidity material without damaging the flexibility of the other part 15. But the cutting part 14 and the other part 15 may be manufactured as one united body.

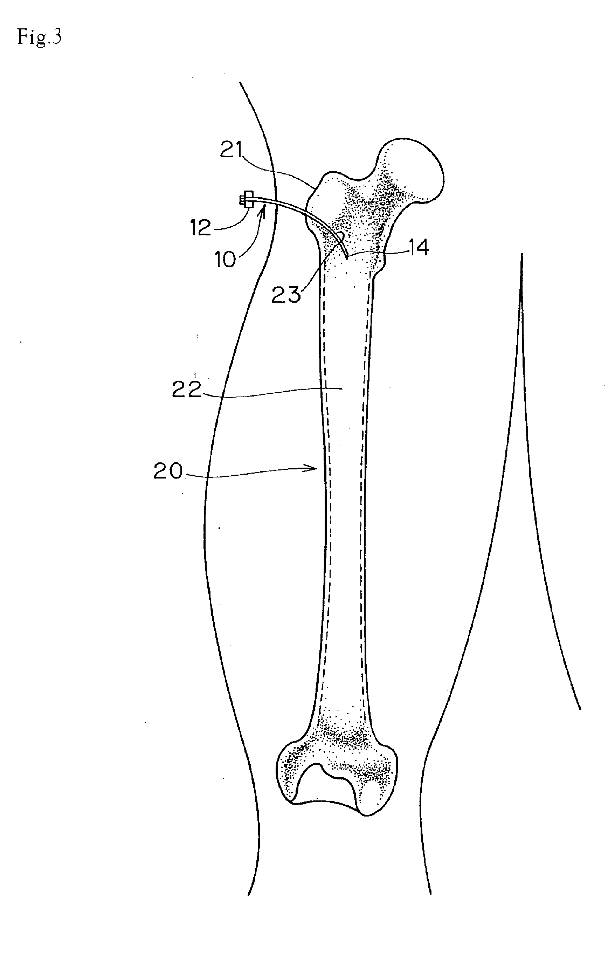

The above puncturing needle 10 is, as shown in Figure 3, stuck to the greater trochanter 21 of the femur (long tubular bone) 20 of donors or patients and pushed forward in the direction of the medullary cavity 22. The operator clasps the grip 21, and pushes and pulls it back and forth or twist from side to side alternately so as to perforate a hole 23 with front edge of the cutting part 14 and the cutting blade. The hole 23 presents a bended shape along the bended shape of the puncturing needle 10 wholly, and its diameter is about the same as that of the thick part 14a of the cutting part 14. Therefore, a gap between the hole 23 and the pipe like part 15 is made, and the scraping of bones is discharged to outside through the gap or through the opening 16. The puncturing work can be done seeing through by roentgen rays.

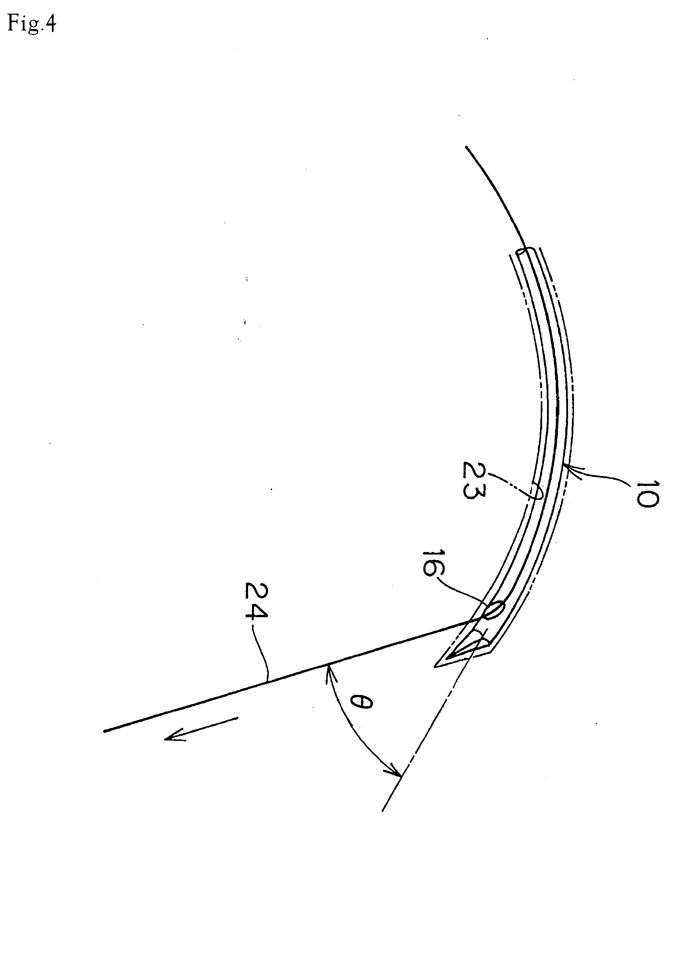

The bended shape of the needle 11 is necessary only at its front edge, and the remaining back part may be made of soft material as far as it can transmit pressurizing force/ tensiltiy or torsional force. In this case, the vicinity of the bended front edge having rigidity is guided by the bended hole 23 which is completed half way. Therefore, if it can transmit the pressurizing force or torsional force, the completed hole 23 and the continuous hole 23 having bended circular arc shape can be extended. The puncturing work may be carried out until the front edge of the puncturing needle 10 reaches the medullary cavity 22. However, the guide wire 24 may be inserted into the cavity 13 and punctured or penetrate the spongy bone 22a with the guide wire 24 in the condition that the needle reaches the spongy bone, as shown in Figure 4.

In this case, as shown in Figure 4, the opening part 16 is provided inside the bending of the puncturing needle 10 and inclined to against the centerline at the angle θ. Therefore, depending on the relation between the condition of the applied force and the passage of the needle, the possibility of bone chips to be pushed into the opening 16 becomes small. Further, even when the curve of the needle is not enough and the front edge is not directed straight forward to the medullary cavity 22, the front edge of the guide wire 24 can be directed to the medullary cavity 22 and advanced to the inside of the spongy bone. Thus, in the case that the puncturing of the spongy bone 22a is carried out by the guide wire 24 from the middle, the hole 23 punctured by the guide wire is thin and straight. But the thin hole 23a can be enlarged and it can be punctured wholly as circular arc by an exploring tube etc.

The guide wire 24 passed through the cavity 13 of the puncturing needle 10 is composed of a publicly known flexible metal wire such as stainless steel wire. The outer diameter of the guide wire 24 is preferable to be smaller than the inner diameter of the cavity 13 so as to be capable of sliding smoothly in the cavity. The front edge of the stainless steel wire is preferable to be hardened. The guide wire 24 works as a guide when an exploring tube or a catheter is passed through the bended hole 23. After the guide wire 24 is passed through, the puncturing needle 10 is taken out. In this case the puncturing needle 10 is easily be extracted by clasping and pulling the grip 12.

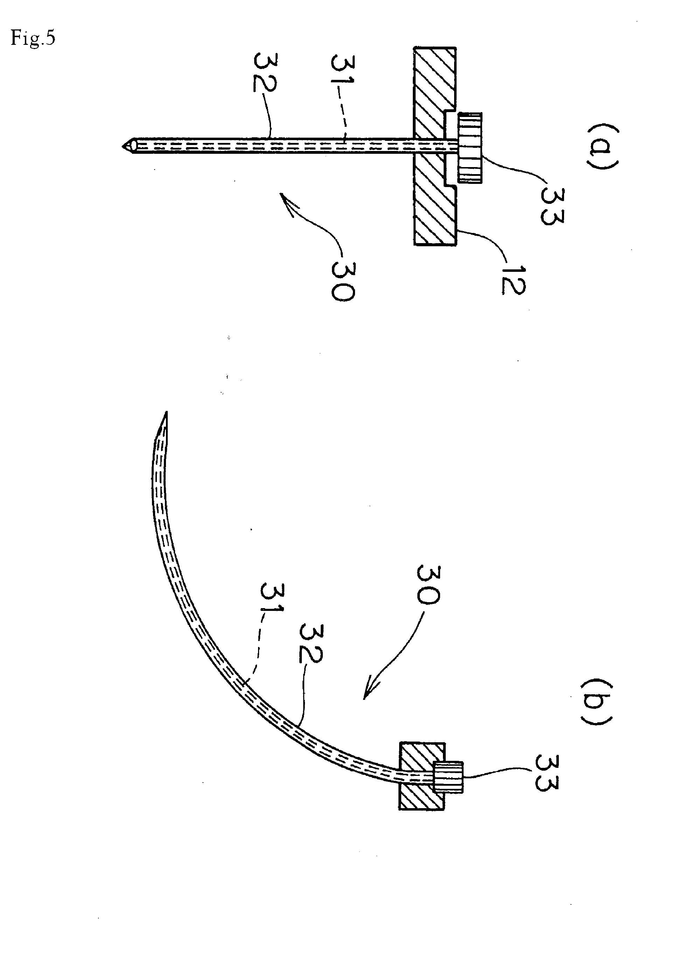

The puncturing needle 30 shown in Figure 5a and Figure 5b have a sheathed core structure provided with an inner needle 31 and a tubular outer needle 32 in which the inner needle is accommodated. The inner needle 31 has flexibility and a knob 33 is fixed to the base edge. On the other hand the outer needle 32 has rigidity and is bended. To the base edge of the outer needle 32, the grip 12 similar to that shown in Figure 1 is fixed. The inner needle 31 is accommodated in the outer needle 32 reciprocably and rotatably. The outer needle 32 is bended but the inner needle 31 has flexibility, so the inner needle can reciprocate widely and continuously rotate in the same direction. The inner needle 31 can be provided movably in the axial direction in the outer needle, but it can also be interlocked so as not to move. The puncturing needle 30 can continue efficient puncture by clasping the grip 12 and reciprocating or rotating the knob 33 while pushing toward the front edge.

The above interlock mechanism is preferable to be capable of changing the status between the condition in which the inner needle 31 is not moved to the near side axially and the condition to be movable. In this case, by switching to the movable condition, the inner needle 31 can be taken out from the outer needle 32 allowing the guide wire to be guided with the outer needle 32 when the guide wire is inserted. The above needle 31 can be composed of the wire rods having high flexibility such as spring steel wires. The outer needle 32 can be composed of metal pipes or tubes such as stainless steel.

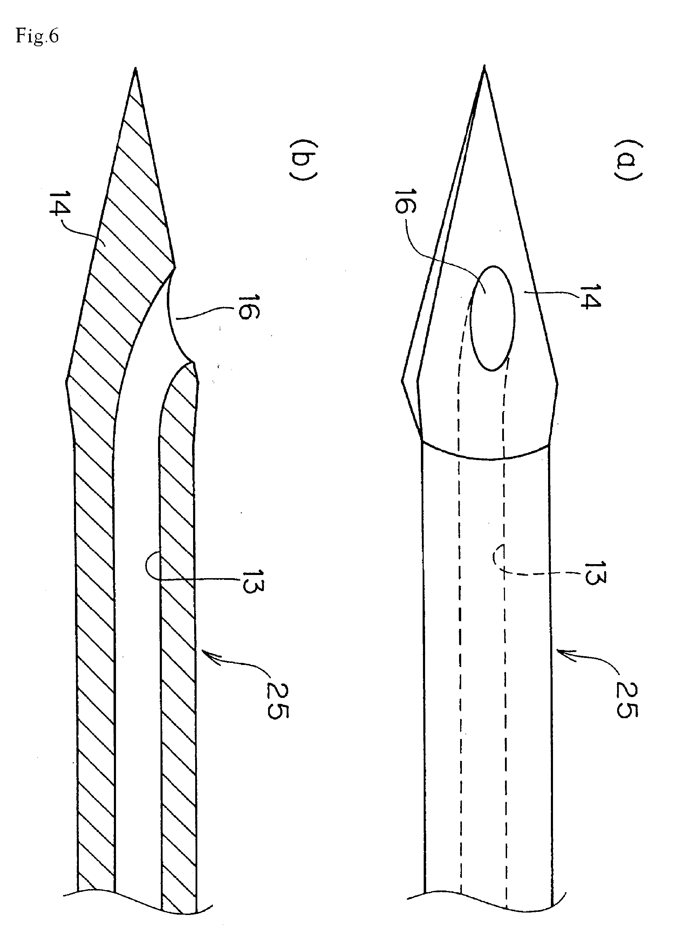

In the puncturing needle 25 shown in Figure 6a and Figure 6b, the opening 16 of the front edge of the cavity 13 is provided on the surface of the cutting part 14. The other part is the same as the puncturing needle 10 shown in Figure 2a and Figure 2b. Since the puncturing needle 25 has the opening 16 provided on the cutting part 14, the bone chips generated by the cutting can be easily discharged from the opening 16 to outside.

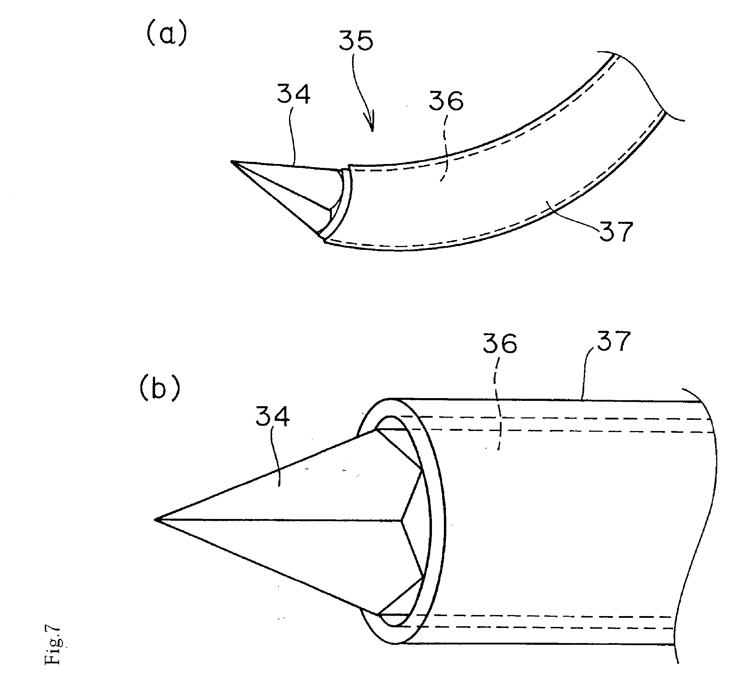

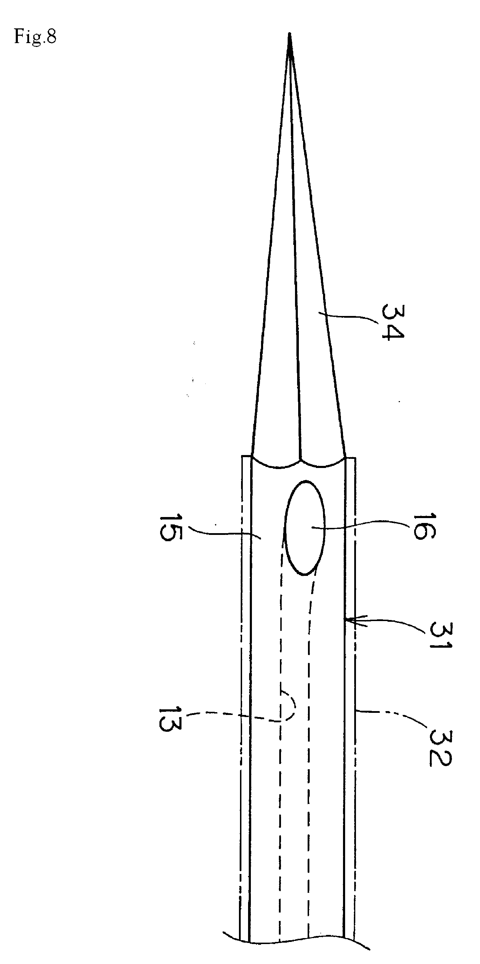

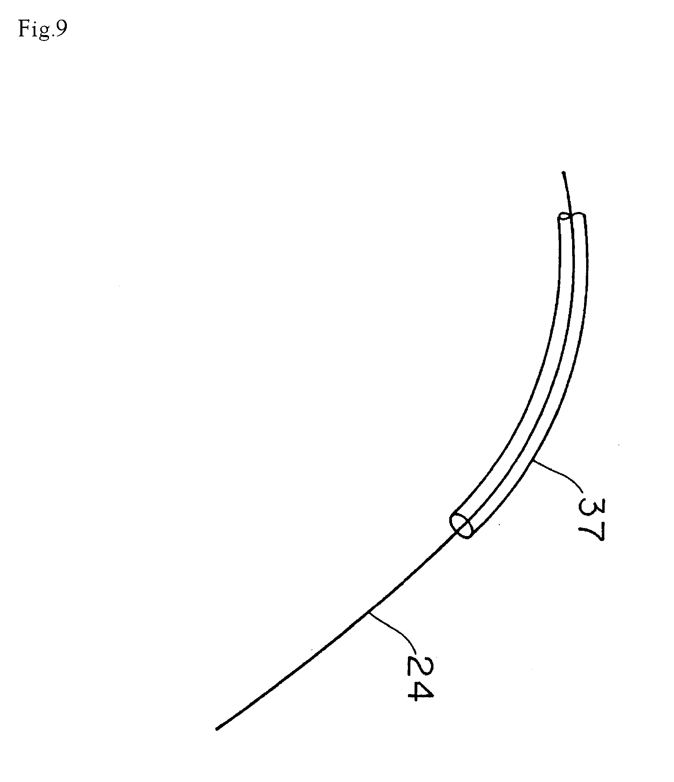

The puncturing needle 35 of Figure 7a is made to be a sheathed core structure provided with a flexible inner needle 36 and a bended outer needle 37 which guides the inner needle. The inner needle 36 is substantially the same as the inner needle 31 shown in Figure 8 later described excepting that the cavity is not provided. More specifically, as shown in Figure 7b, the inner needle 36 has a multi-pyramid cutting part 34 in its front edge and accommodated in the tubular outer needle 37 reciprocably or rotatably. When such inner needle 36 without cavity is used, the inner needle 36 is so composed as to be extractable from the outer needle 35. Thereby, as shown in Figure 9, the guide wire 24 can be passed through or inserted into the outer needle 37 after the inner needle 36 is taken out.

In the inner needle 31 shown in Figure 8, the pyramid cutting part 34 same as the cutting part 14 shown in Figure 2 is provided on its front edge. But in this cutting part 34, the thick part (see Reference numeral 14a of Figure 2a) is not provided, and is composed of a plurality of the plane tapered toward the front edge from the continuing part 15 with the same diameter. Therefore, the insertion to the outer needle 32 from the front edge side is easy. The provision of such cutting part 34 enables to puncture bones by reciprocating or rotating the inner needle 31 in the outer needle 32. Further, the inner needle 31 has a cavity 13 extending axially same as the puncturing needle 10 shown in Figure 2a. The cavity 13 is opened laterally at a somewhat backward position from the cutting part 34. Therefore, the cut bone chips can be discharged through the cavity 13.

In the case that a rigid material is used as a material for the inner needle 31, and that it is so composed as to have a bended shape, it can be used as an puncturing needle itself similarly to the case in Figure 2a and Figure 2b. Further, Such a needle in which flexible tubular outer needle 32 is loaded around the periphery of the inner needle 31 having rigidity can be used as a puncturing needle. In this case, a cutting blade can be provided on the front edge of the outer needle and it can be advanced rotating the outer needle 32 with the guide of the inner needle 31. The puncturing needle having a sheathed core structure of this invention includes such a needle of which the inner needle has rigidity and the outer needle has flexibility.

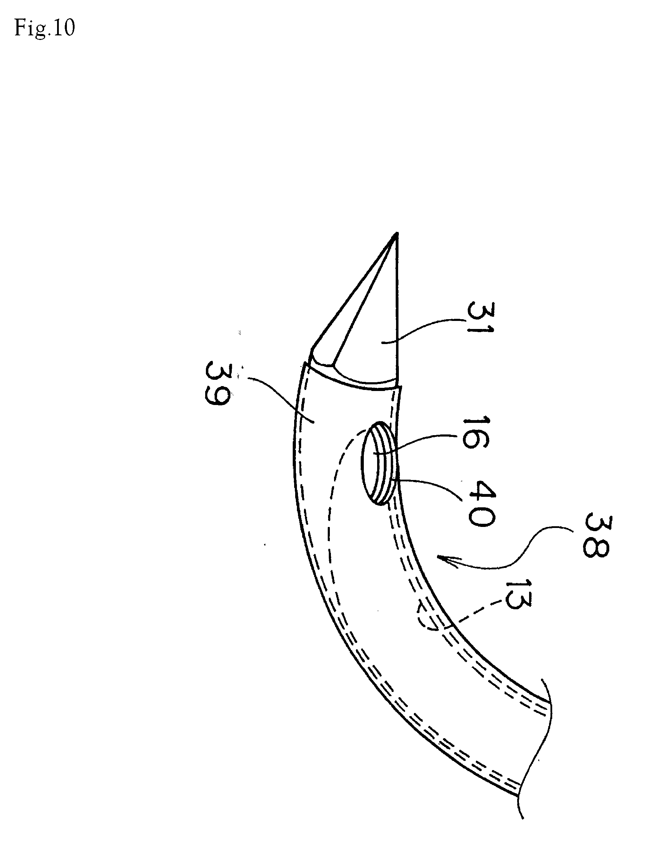

The puncturing needle 38 of Figure 10 is provided with the sheathed core structure composed of the inner needle 31 and the outer needle 39. The inner needle 31 is provided with the cavity 13 and the lateral opening 16, and the outer needle 39 is provided with the opening 40 laterally. When the opening 16 of the inner needle 31 and the opening 40 of the outer needle are lined up, the cavity 13 communicates with outside via the opening 16, 40. In this condition, similarly to the case in Figure 4, the front edge of the guide wire can be led out from the front side opening 16, 40 by inserting the guide wire from the base edge side.

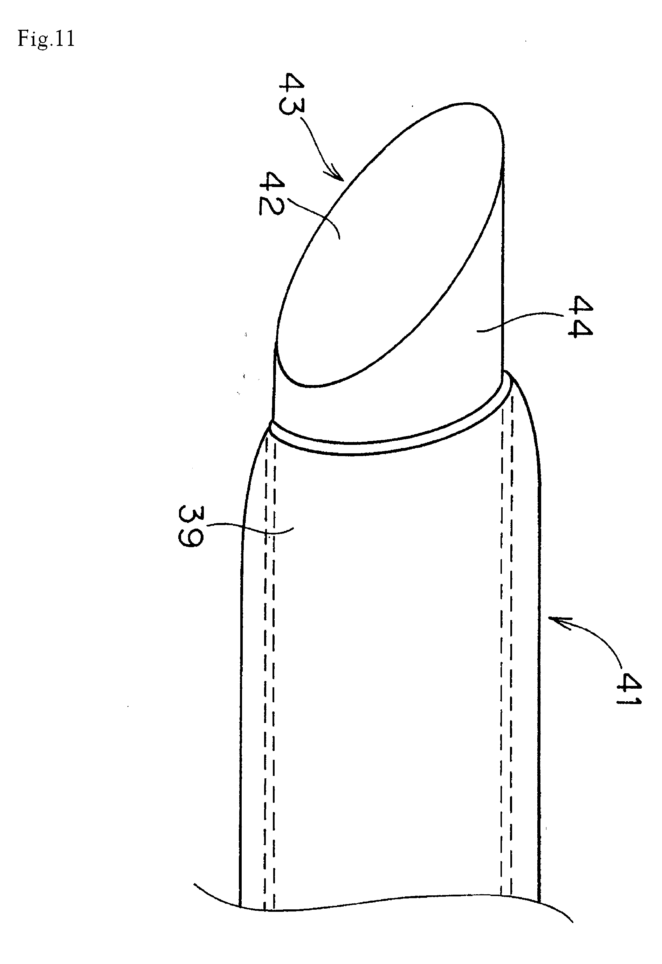

The puncturing needle 41 of Figure 11 is provided with an inner needle 44 having a cutting part 43 of which the shape is such that a column-shaped wire rod is cut by an inclined plane 42, and an outer needle 39 provided around the periphery of the inner needle 44. Thus, the shape of the cutting part of the inner needle is not limited to those of multi-pyramid but it may be that the cutting blade is composed between the one plane of the inclined plane 42 and the ridgeline of the periphery plane. Even such cutting part 43 can puncture bones by rotating or reciprocating.

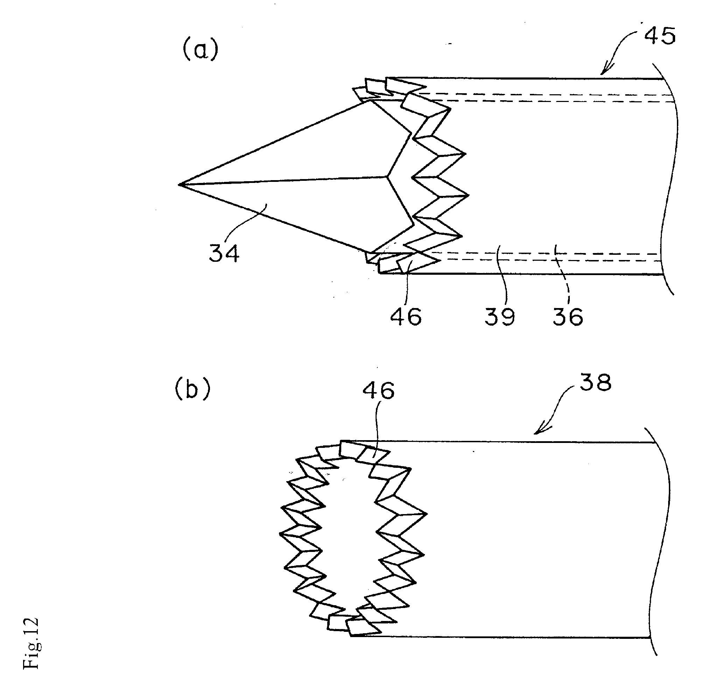

The puncturing needle 45 of Figure 12a is the same as the puncturing needle of Figure 7b excepting that the front edge of the outer needle 39 of the cutting part 46 is formed in sawtooth. The cutting part 46 is composed of a plurality of triangular teeth aligned annularly like shown in Figure 12b. The number of the teeth is not particularly restricted, but usually three or four each is sufficient. When the outer needle 39 is rotated or reciprocated, a circular groove can be cut. This can puncture with the cutting part 34 of the inner needle 36 by reciprocating or rotating in one direction, and further, can cut the annular groove corresponding to the cross section of the cutting part 46 of the outer needle 39 by slightly reciprocating the outer needle 39. Thereby allowing efficient puncturing. As described above, even when the bended wire rod having rigidity is used as the inner needle and a tubular outer needle 39 having flexibility is provided on its periphery, the provision of such sawtooth cutting part 46 facilitates efficient puncturing.

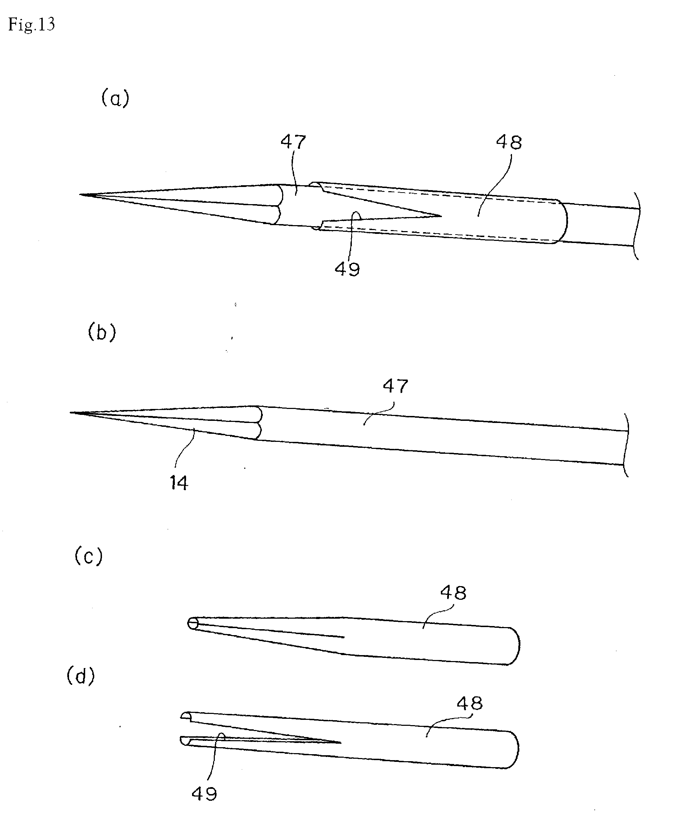

The puncturing needle 47 shown in Figure 13a is provided with a sleeve 48 slidably around its periphery. In the front edge of the sleeve 48, a V-shaped notched part 49 is formed. The puncturing needle 47 has rigidity, similar to the puncturing needle of Figure 1 etc. The puncturing needle 47 is a wire rod of which the cross section is circular, having cone cutting part 14 in the front edge (see Figure 13b). The notched part 49 of the sleeve 48 has an opening/closing mechanism to open as shown in Figure 13d when the puncturing needle 47 protrudes as shown in Figure 13a, and to close as shown in Figure 13d when the puncturing needle 47 is kept back as shown in Figure 13c. Additionally, the closing action is preferable to be done by the elasticity of the front edge of the sleeve 48. As the sleeve 48, a material having elasticity, for example, synthetic resin etc. is used. But it can be made of thin metal etc. The provision of such opening/closing mechanism makes it hard for the front edge of the puncturing needle 47 to touch the skin and to be contaminated by bacteria of the skin surface, prevents the bacteria to enter the body in the many times insertion work of the puncturing needle. Therefore it prevents from the infection.

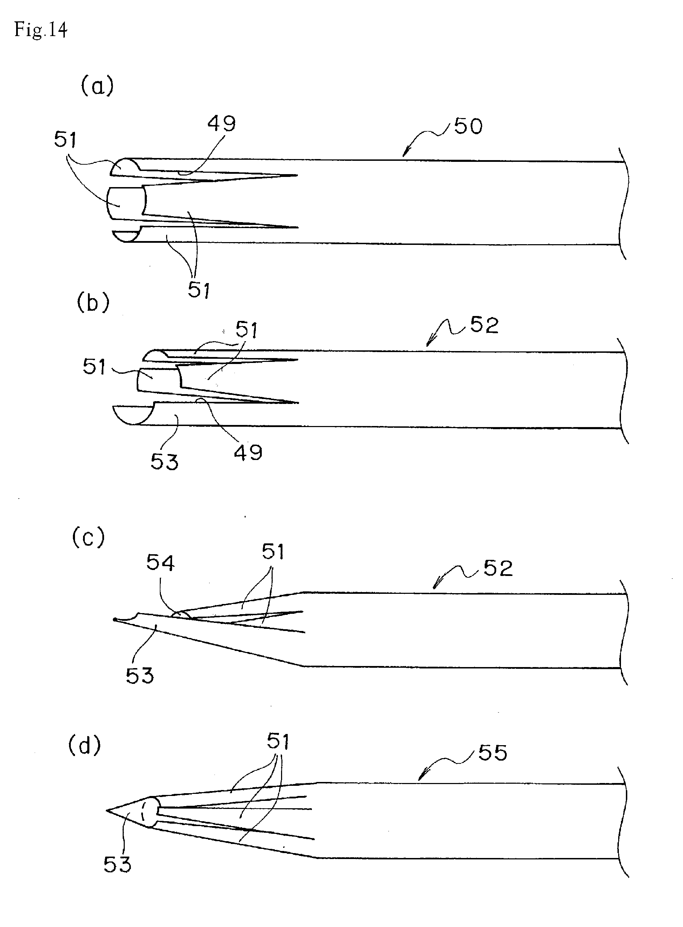

Figure 14a shows a sleeve 50 having four notched part 49. This facilitates the opening/closing action of the movable portion 51. In the sleeve 52 shown in Figure 14b, four notched parts are formed and one of the movable portions 53 is longer than the other movable portions 51. This forms an opening 54 laterally (upper side in the figure) when the movable portion 51, 53 close as shown in Figure 14c. Therefore, when the guide wire is guided while changing direction laterally, bacteria are hard to enter from the gap. In the sleeve 55 shown in Figure 14d, the front edge of the long movable portion is sharpened. This sleeve 55 is easy to be left in the hole punctured by the inner hole when the inner needle is taken out, and easy to return to the original position when it is misaligned back and forth in the hole. Furthermore, it has a merit that the opened hole is easily located when it drops out form the bone by any possibility.

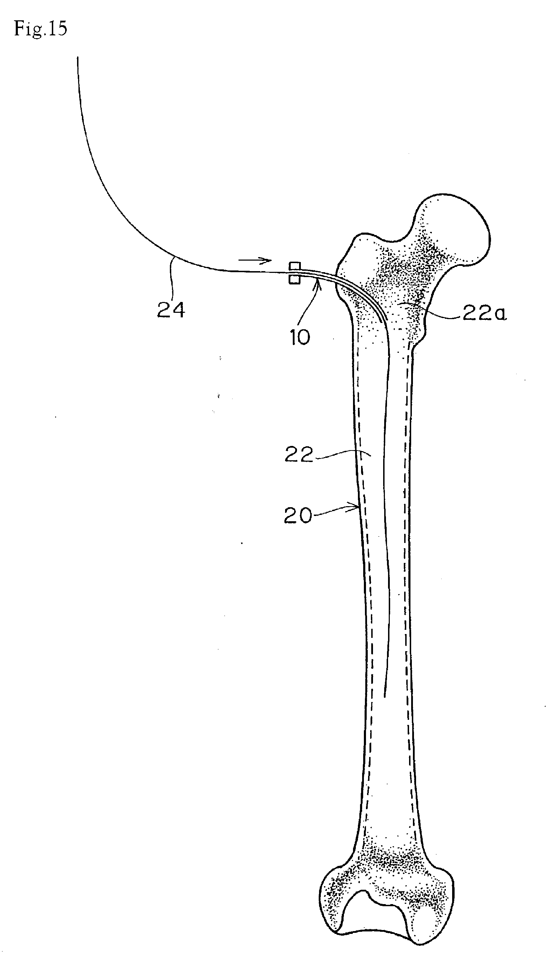

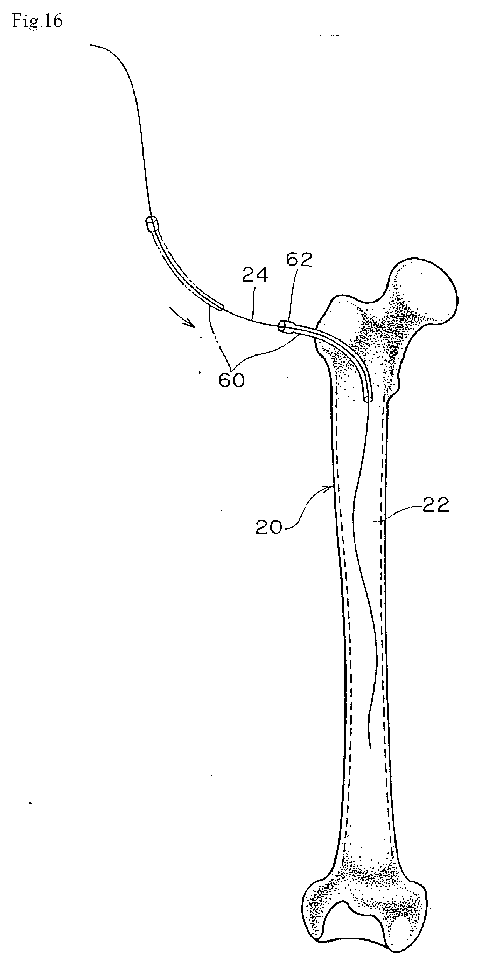

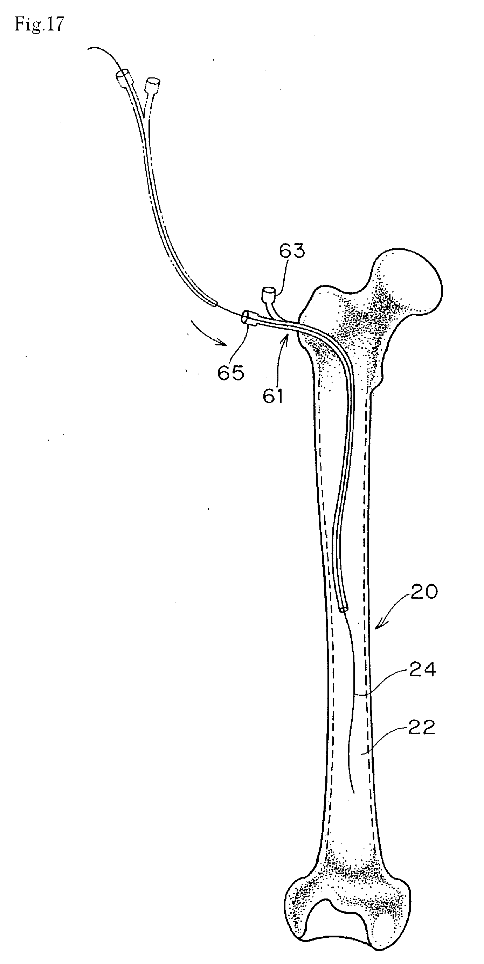

Next, the method for harvesting the bone marrow of a long tubular bone using the puncturing needle 10 of Figure 1 is described. This method is also an embodiment of the method for harvesting the bone marrow of this invention. At first it is described schematically. As shown in Figure 3, In the epiphysis of the tubular bone 20, the thinly curved puncturing needle 10 is stuck into the point where it is out of arthrosis region and easy to puncture, and the puncturing needle 10 is advanced into the spongy bone from the punctured point. Then, the front edge of the puncturing needle 10 is directed in the spongy to the medullary cavity 22a, being advanced as it is, or after reaching the spongy bone, as shown in Figure 15, the guide wire 24 is passed through. And then, the front edge of the guide wire penetrates the spongy bone to reach the medullary cavity 22. Further, the puncturing needle 10 is taken out leaving the guide wire 24, as shown in Figure 16, the exploring tube 60 is loaded along the guide wire 24 to secure the path reaching the medullary cavity 22. A sample of the bone marrow is harvested through the exploring tube 60. A large amount of the bone marrow is harvested by loading the catheter like shown in Figure 17 to 19. This invention provides a method to harvest the bone marrow easily and securely from the tubular bone, and provides a kit facilitating the method by combining the curved puncturing needle 10 and the pertaining catheter 61 etc.

The large difference between this invention and conventional art is the employment of the method in which the curved thin puncturing needle is stuck from the side apart from the arthrosis region of the epiphysis of the tubular bone and the front edge of the needle is directed to the bone marrow in the bone. In other words, when a conventional straight puncturing needle was used, it was necessary to insert axially from the epiphysis or to puncture aslant from the vicinity of the epiphysis. However because there is an arthrosis in the epiphysis and the long axis of the bone lies in the same direction of the body, the puncturing in the axis direction was difficult. Since this invention uses the bended puncturing needle, it is possible to puncture in the right angle or a near angle to the side in the epiphysis, thereby the puncturing is easy and the burden imposed on donors and patients is light.

The cortical bone existing on the bone surface of epiphysis is thinner than the compact layer of the diaphysis. Nevertheless it is the end part of the bone, so the cortex of bone is hard and difficult to puncture. Therefore, in this invention, the front edge of the puncturing needle has the sharp cone structure to break the cortical bone easily. Meanwhile, in many prior arts, the front edge of the puncturing needle was sharpened to break the cortical bone by strongly sticking the needle into the bone. Therefore, accidents were reported, in which an excess force was applied to the bone surface resulting in slip of the needlepoint causing the deep sting of soft tissues. Therefore, in many prior arts, a stopper for safety device has been conceived. However, because of the reason that small forces are required in screwing than strongly pushing in with all one's strength, the Patent Publication 3 is reported, in which the front edge has a drill structure. When this method is used, it is possible to advance the puncturing needle without applying a force in the direction of long axis, and it is safe. In regard to this, in this invention a cone cutting part is provided on the front edge of the puncturing needle. Thereby it is possible to puncture the hard cortical bone without applying excess force by rotating the puncturing needle. This composition eliminates the need for excess forces and a safety device to be incorporated for preventing excess insertion.

Further, in the method of this invention, since the object bone of harvesting the bone marrow is larger and thicker than that of conventional arts. There is fewer possibility of penetrating the bone when just pushing by the force. In addition, there are few important organs around such bones, and because the bones are surrounded by large muscles, even when the puncturing needle slips and stings the surrounding organs (including skins), real damages caused by the side injury are few.

As described above, the operation of this invention is carried out by breaking the skin by the puncturing needle and advancing the front edge of the puncturing needle into the spongy bone. However the thickness of the spongy bone is largely different depending on the personal difference, age difference, sex difference, further the difference of exercise habit, living habit. Accordingly, in a person whose spongy bone layer is thin, the front edge of the puncturing needle happens to reach the medullary cavity, but in many cases, it is not possible. Therefore, as described above, the guide wire is inserted into the cavity of the puncturing needle, or when it is of double structure, the inner needle is taken out and the guide wire is inserted into the outer needle to puncture the spongy bone to reach the medullary cavity.

Fortunately, the spongy bone has a structure to be fully penetrated by such a degree of the guide wire. There is a small resistance, while penetrating, but the resistance subsides abruptly at a point where it enters into the medullary cavity allowing the guide wire to advance easily. The front edge of the guide wire will be stopped by touching the inside of the epiphysis of the opposite side by further advancing the guide wire. And because the position of the stop can determined by the length of the bone, it is easily estimated. If it is insertable beyond the estimation, the operator should conceive that the guide wire is not inserted into the bone.

Since the above guide wire needs to advance in the spongy bone, some degree of hardness in the front edge is required. But the guide wire also needs to explore the epiphysis, excess stimulus is not preferable. Therefore, it is convenient to make other end to be flexible for such uses.

A force is needed to puncture the puncturing needle into a cortical bone, and also a large force is needed for the needle to break the cortical bone and to go out of the bone. Therefore, usually the front edge of the guide wire which entered into the medullary cavity by once breaking the cortical bone never goes out of the bone again. If it goes out, it is the result of advancing the guide wire in the stage of not breaking the cortical bone from the beginning. Therefore, considerations are required to puncture the cortical bone securely not to result in such a case. For this ascertainment, it is preferable to see through using roentgen rays.

When the guide wire 24 reaches the medullary cavity 22 as described above, as shown in Figure 16, the puncturing needle 10 is taken out and in place of it the exploring tube 60 is advanced in the spongy bone with the guide of the guide wire 24. The exploring tube 60 is a tubular member having such bendability as to be capable of advancing in the curve along the guide wire 24. Particularly such bendability as to be capable of advancing from the curved hole formed by the puncturing needle 10 (see reference numeral 23 of Figure 4) to the straight thin hole formed by the guide wire 24 is provided. The front edge is not always required to have such sharp front edge structure as that required for the puncturing needle, but the hardness to be capable of advancing in the spongy bone is required. Further, the base edge is preferable to be provided with a means to aspirate the sample of the bone marrow, for example, a configuration to be capable of connecting an injection syringe. In the case of Figure 16, a cylindrical portion 62 having an enlarged diameter is provided as the junction. Such guide wire 24 may be made of flexible metal or may be made of synthetic resin.

Next, at the point where the exploring tube 60 is inserted into the bone about 10cm, the guide wire 24 is taken out, and an injection syringe is attached to the base edge of the exploring tube 60 to aspirate for testing whether the bone marrow can be aspirated or not. If the bone marrow were not aspirated, the exploring tube 60 is further advanced along the guide wire 24 to the point where the bone marrow can be aspirated. If any bone marrow cannot be aspirated at the point where the exploring tube is advanced more than 30cm, the harvesting is given up as no bone marrow exists in the bone. But even in the case of aged person, the insertion less than 15cm can achieve the level capable of harvesting the bone marrow in many cases. This operation is a pure intraluminal dilatation work in the spongy bone by the exploring tube 60.

When the exploring tube 60 reaches the medullary cavity 22 as described above, the guide wire 24 is passed through the exploring tube again. The exploring tube 60 is taken out leaving the guide wire and alternatively the catheter 61 shown in Figure 17 is inserted. In order to facilitate this operation, the thickness of the puncturing needle 10, the exploring tube 60, and the catheter 61 is preferable to be the same. Usually, it is advantageous to make it be as large as the instillator the size of which is 12 gauges to 15 gauges, more specifically, the inner diameter is about 1.4mm to 2.4mm and the outer diameter is about 1.8mm to 2.8mm. In the expression of french, the catheter of the size of 5 french to 8 french can easily harvest the bone marrow. Such a technique of using the catheter is a concept of diverting the Seldinger's catheter technique, and the same material and composition of the catheter for use of blood vessels can be used. As for Seldinger's catheter technique, for example, a part of the technique is described in the Patent Publication 5, and it is possible to divert the concept of existing techniques.

Usually in the case that a catheter is inserted into a blood vessel by Seldinger's catheter technique, a catheter which is thicker than the puncturing needle used at first along the guide wire can be inserted, because the vessel wall is soft. However, when a catheter is inserted into a bone, such thick catheter as used for blood vessels cannot be inserted. It is difficult to insert a catheter thicker than the size of the punctured hole, because at least the cortical bone is hard. Therefore, in the case of the catheter 61 shown in Figure 17, as described above, it is important to secure the through hole of required size prior to the use of the exploring tube, and the thickness of the catheter is adapted to the hole size.

The catheter 61 not only aspirates the bone marrow, but also is used as a path to inject a dilute solution such as a physiological salt solution etc. in order to make the liquid of suspended particle of the bone marrow in the medullary cavity 22. For that purpose, the catheter 61 has a configuration in which the base edge branches out into two. The one junction 63 is connected to an infusion solution bag 64 charged with a physiological salt solution, and another junction 65 is connected to an injection syringe 66 for aspiration. In each junction, a three way turncocks 67, 67 for switching are provided. Thereby, even it is one catheter 61, it can inject the physiological salt solution from the infusion solution bag 64 switching the three way turncock 67, 67 and can aspirate the liquid of suspended particle of the bone marrow by the injection from the syringe 66.

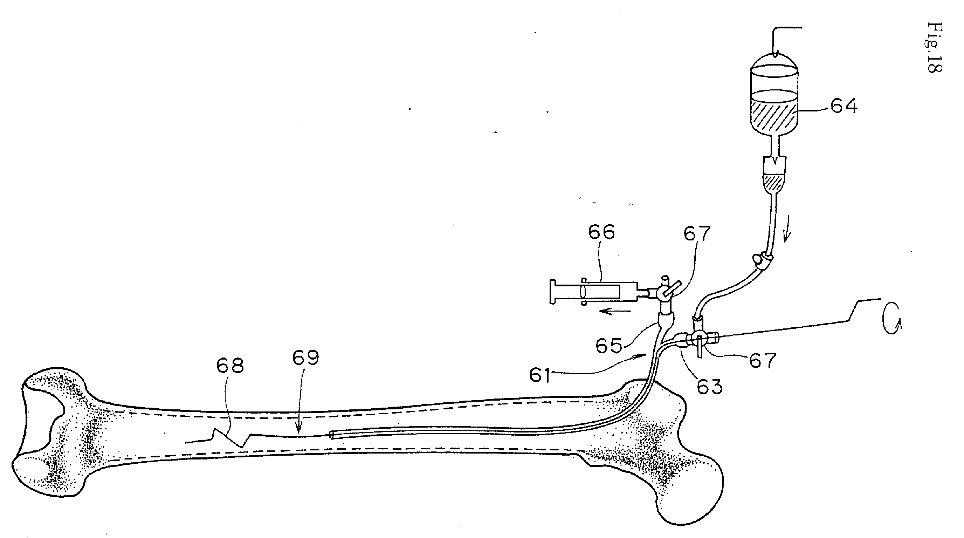

In the medical apparatus of Figure 18, a guide wire 69 provided with a bended stirring part 68 at its front edge is inserted into or passed through the catheter 61. The guide wire 69 of which the front edge is bended can be composed of the metal guide wire same as common guide wires, but the straight guide wire for puncturing the spongy bone is made to be a separate guide wire. When such guide wire 69 provided with the stirring part 68 is rotated to stir inside of the medullary cavity 22, it is possible to shave and get the bone marrow in every corner of the medullary cavity, and simultaneously it is possible to produce the liquid of suspended particle of the bone marrow in the bone marrow cavity. Therefore, it allows even a thin catheter to harvest easily suppressing the interfusion of the peripheral blood of the bone marrow. For example, synthetic resins such as polyethylene, polyamide (nylon), polyurethane, tetrafluoroethylene (Teflon (registered trade mark)) are used. Further, such metals as stainless steel, titanium alloy, cobalt alloy, shape-memory alloy etc. can be used.

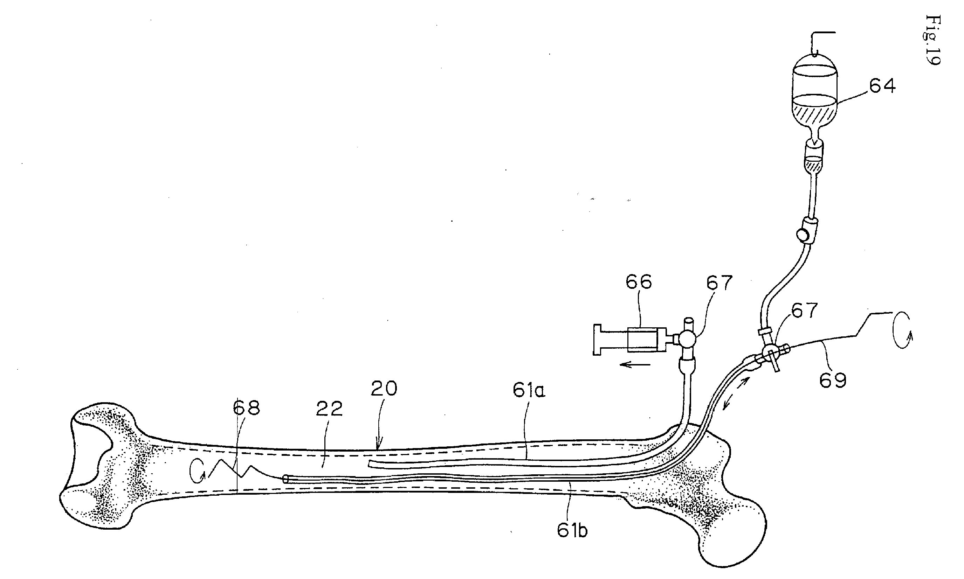

Since, as described above, the catheter 61 injects the physiological salt solution and aspirates the liquid of suspended particle of the bone marrow at the same time, as shown in Figure 19, inserting two catheters 61a, 61b or using the multi-lumen such as double lumen etc. facilitates the operation. For example, in the case shown in Figure 18, a catheter provided with three lumens; a lumen for injecting the physiological salt solution; a lumen for aspirating the liquid of suspended particle of the bone marrow; and a lumen for passing through the guide wire 69 for stirring is preferable. However it is preferable not to puncture a large hole to the bone even when the a catheter having such structure is inserted. Therefore, it is preferable to insert thin catheter additionally, to share the role (see Figure 19).

In order to satisfy these conditions, it is required to use the thin catheter for reducing the burden of patients. The front edge diameter of femur of human and large mammal is about 2cm and tendons of peripheral muscles are attached in this region. Therefore, puncturing a large hole to this region may destroy the bone structure causing disabilities in the future, and at the same time, leaving possibly continuous pain due to the delay of the cure of the bone punctured. Therefore, the thickness of the catheter is preferable to be as thin as possible, for example, to be less than 6mm in the outer diameter, preferably less than 4mm, further less than 3mm and furthermore less than 2mm.

As the size of the catheter, the inner diameter of 1.5mm is sufficient for aspirating the bone marrow. However, since it happens to pull in small bone chips in the medullary cavity, the larger thickness is advantageous. But even in the double lumen structure, the outer diameter is preferable to be less than 6mm as described above and is preferable to be thin as far as possible. Therefore, it is preferable to be made of polymeric material to earn the inner diameter much.

As shown in Figure 18, in the case that the physiological salt solution is injected from the infusion solution bag 64, the liquid is stirred by the guide wire 69, and simultaneously the liquid of suspended particulate of the bone marrow is aspirated by the injection syringe 66, it is important to fill up the physiological salt solution in the catheter 61. It is because, traffic to the peripheral blood vessel is densely formed in the bone marrow cavity, the existence of air in the catheter causes possibly the air to be sucked in into the blood vessels. In order to satisfy this condition a structure capable of connecting the injection syringe 66 at the near side of the catheter 61 is provided in the apparatus shown in Figure 18. And it is connected via the three-way turncock 67 which is in heavy usage in medical fields. Further, it is preferable to joint an adverse current check valve used in the catheter operation of blood in its front edge. Thereby, the structure for passing through the guide wire can be maintained preventing the leakage of the physiological salt solution.

The medullary cavity is not of a simple tubular shape. The central part of the medullary cavity is crowded with the bone marrow having a hardness of the degree of agar gel, and the inside surface of the medullary cavity is not smooth with complicated protrusion of bone tissues forming labyrinth structure. In each of the labyrinth is also filled up with the bone marrow. In order to scrape and draw them as much as possible, it is impossible to do only by aspiration. In the above described non patent literature 3, it is described that aspiration of the agar was possible only at the portion where the hard catheter is passed through. A simple aspiration can aspirate only a small amount of the bone marrow. Even if the circulation method is used at the same time, because the bone marrow has a degree of hardness, the aspiration is not easy, and it appears to be reality that the amount of harvesting is limited.

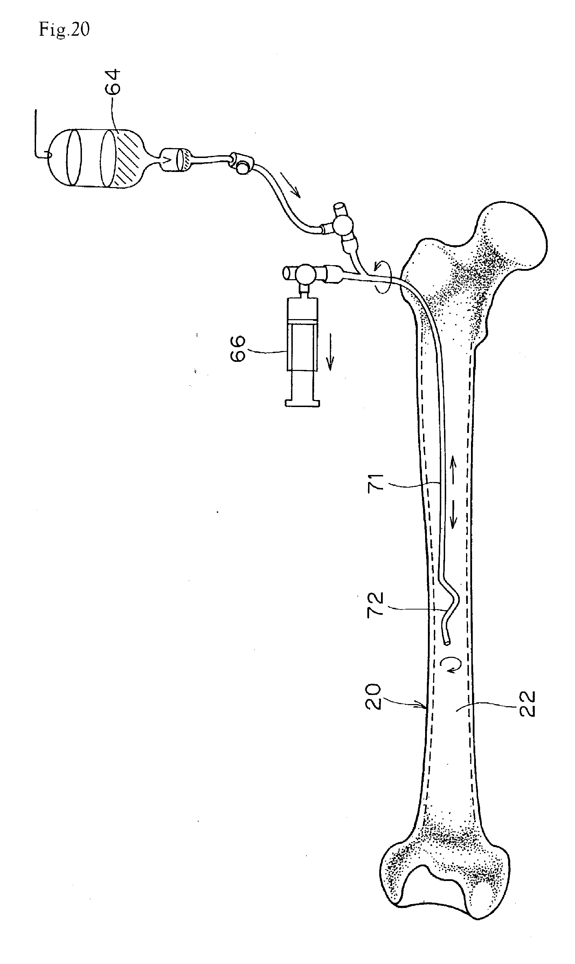

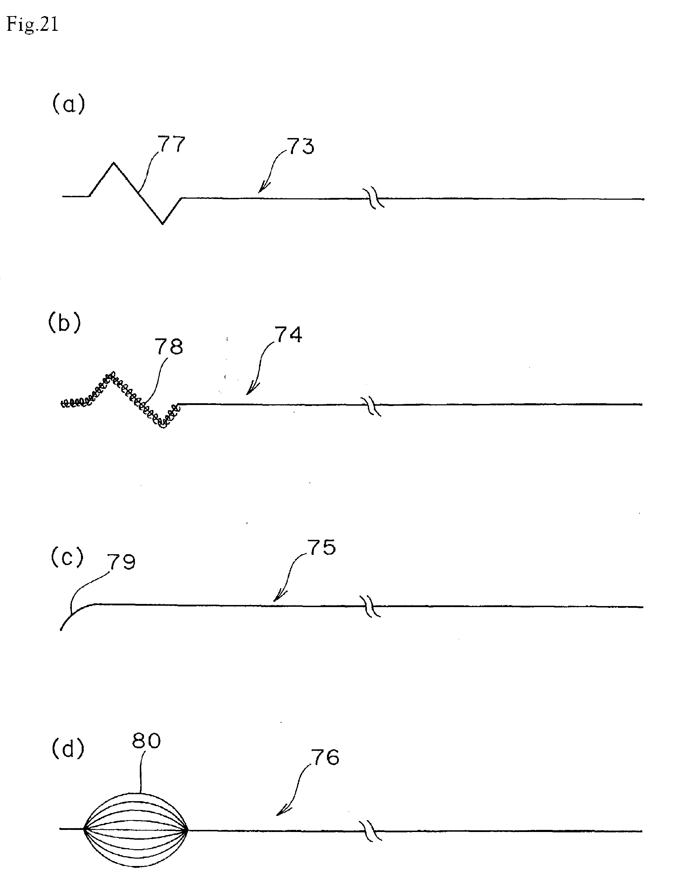

In the medical apparatus shown in Figure 20, a stirring part 72 is provided by bending the front edge of the catheter 71 itself. Thereby, rotation or reciprocation of the catheter 71 stirs of the medullary cavity and increases the harvesting rate. For further increase of the harvesting rate, the use of stirring wire 73-76 for exclusive use is recommended as shown in Figure 21 a to 21 d. More specifically, it is preferable to use a stirring wire 73 (see Figure 21a) provided with a bended portion 77 in its front edge, a stirring wire 74 provided with a coil like brush structure 78 in its bended portion, a stirring part 75 provided with a bended portion 79, and a stirring part 76 having multi-filament composition, in which it is disjointed into mono-filament and expanded into basket like structure 80. By rotating or reciprocating these exclusive use stirring wires 73-76 in the bone marrow cavity, the bone marrow tissues can be fully stirred. And injecting the physiological salt solution at the same time can efficiently make the liquid of suspended particulate of the bone marrow tissue in the medullary cavity. Thereby, the harvesting rate of the bone marrow can be greatly elevated. In addition, when transplanted to patients or object animals of the treatment together with the physiological salt solution, the harvested bone marrow as the mixed solution can be transplanted without change or being adjusted its degree of dilution. Further, it can be stored after the separation of the bone marrow and the physiological salt solution by centrifugal separation.

In the bone marrow harvesting method of Figure 19, two holes are formed in one end of a long tubular bone using the above described puncturing needle, a guide wire, and an exploring tube. A physiological salt solution is injected from one of the catheter inserted in one hole, and the bone marrow is aspirated from another catheter inserted in another hole. In this case, one of the holes can be formed in the lower end of the long tubular bone. However, it is preferable not to employ the circulating method in which a liquid which is once taken out side is returned, and to employ the method to always inject a new liquid. Thereby, inside of the bone marrow is kept clean reducing the opportunities of infection.

The exclusive use stirring wire 73-76 shown in Figure 21 is, as shown in Figure 18 and Figure 19, advantageous to be inserted via a lumen for injecting a physiological salt solution or a catheter. But, taking the role in account, the use of a catheter having three holes lumen is possible, and three thin catheters can also be inserted.

The artifice making the tube have a bend for harvesting the bone marrow is described in the Patent Publication 6. According to this, it is described that the efficient harvesting of tissues is possible by advancing a tube having bended front edge. In the case of Figure 18, Figure 19, Figure 20, and Figure 21, by making not only have a bend but also enables the rotation, the bone marrow of bones is peeled off from the inner wall of the medullary cavity to facilitate the aspiration and harvesting. By injecting the physiological salt solution from the front edge of the catheter simultaneously, it is possible to make the liquid of suspended particulate and the soil suspension of cells of the bone marrow. Such configurations facilitate the aspiration of the bone marrow from the bone marrow cavity even by a simple aspiration.

In the case of Figure 20, a bend is provided in the catheter itself. The degree of the bend is sufficient to be the required minimum of curve and size for stirring the cavity. Specifically, the provision of the bend having about 120 degrees curve positioned one by one respectively at about 1cm, 2.5cm, 4cm from the front edge of the catheter, where the total number is three, makes it possible to stir the bone marrow cavity and the efficient producing of the liquid of suspended particulate of the bone marrow by rotating or reciprocating the catheter in the bone marrow cavity. The bends are preferable to be located at 1 to 5 positions.

Regarding the structure of the exclusive use stirring wire, the similar bend as the catheter (see Figure 21a) may be incorporated. Further, it is conceivable to attach a brush raised with metal or polymer fabric etc. However, taking in account the danger of these brush pieces to drop out in the medullary cavity, the structure in which they drop out by no means must be selected. In such a condition, even a degree of the structure in which the front edge of the wire of the multi-filament is separated into monofilament (see Figure 21d) can sufficiently display its greatest force for the bone marrow stirring. When the degree of stirring is small, to perform the same operation repeatedly increases the stirring effect. Further, by providing the shape-memory characteristics in the front edge, such a structure can be prepared that in the insertion process the front edge is shaped into one wire and exerts the brush effect at the temperature same as the body temperature(around 37 degree Celsius) For example as shown in Figure 21 d, at a given temperature, the multi filament separate into each monofilament to expand presenting basket like shape and at a low temperature, it is shaped into single rod.

The wires and catheters used for these purpose are required to be capable of rotating their front edge in tune with the rotation at the near side. Therefore, it is necessary to select a material excellent in torque transmissibility. The catheter is required to be bendable and flexible, because its inserted part is curved and the diaphysis is not straight and curved. Hard or rigid pipe as stainless steel pipe cannot be employed. As a material for the catheter and the stirring wire, it is necessary to select a quality of material hard to become twisted, convolved so as to prevent them from impossible situation to be taken out due to the twisting or convolving of its front edge while rotated at the near side.

Even being regardful of those, when any resistance is felt in taking out, it is preferable to see through the condition of the catheter and the front edge of the wire by roentgen rays. In order to realize this easily, it is preferable for its front edge to use the material having high projective characteristics of roentgen rays or to be coated. If any problem is found, it is necessary to consult an orthopedic surgeon without hesitation. Even in this time, it is recommended to fill up all inserted catheters with the physiological salt solution. Air must not be inlet to the medullary cavity. Therefore, even in the stirring device, it is preferable to operate according to the general catheter operating procedures with an adverse current check valve being attached. In the above bone marrow harvesting procedure, it is possible to obtain the bone marrow liquid from long tubular bones by above mentioned puncturing needle and the combination of the exploring tube, the stirring wire, and the catheter.

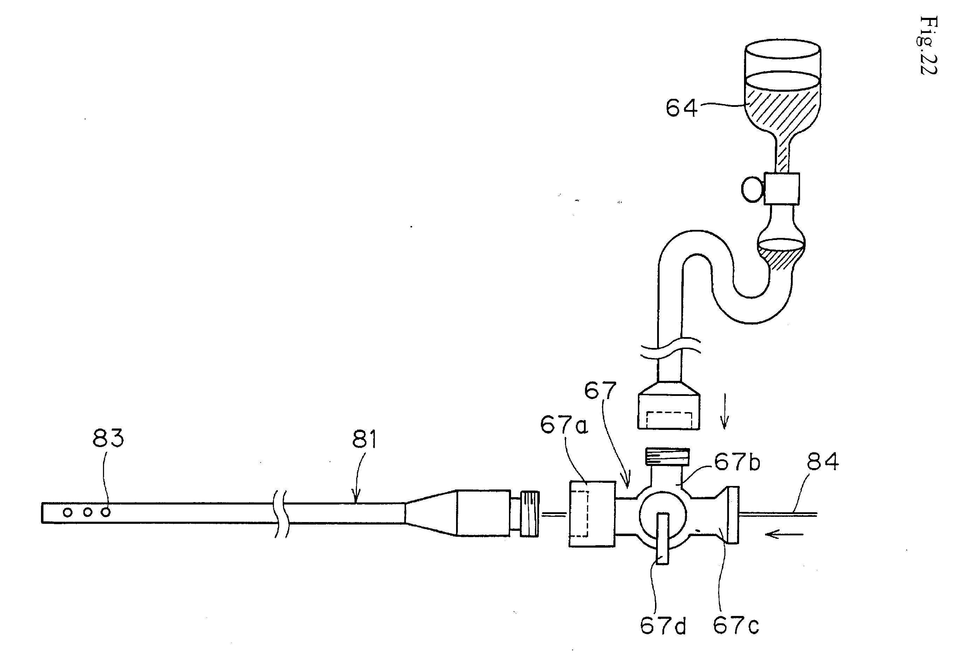

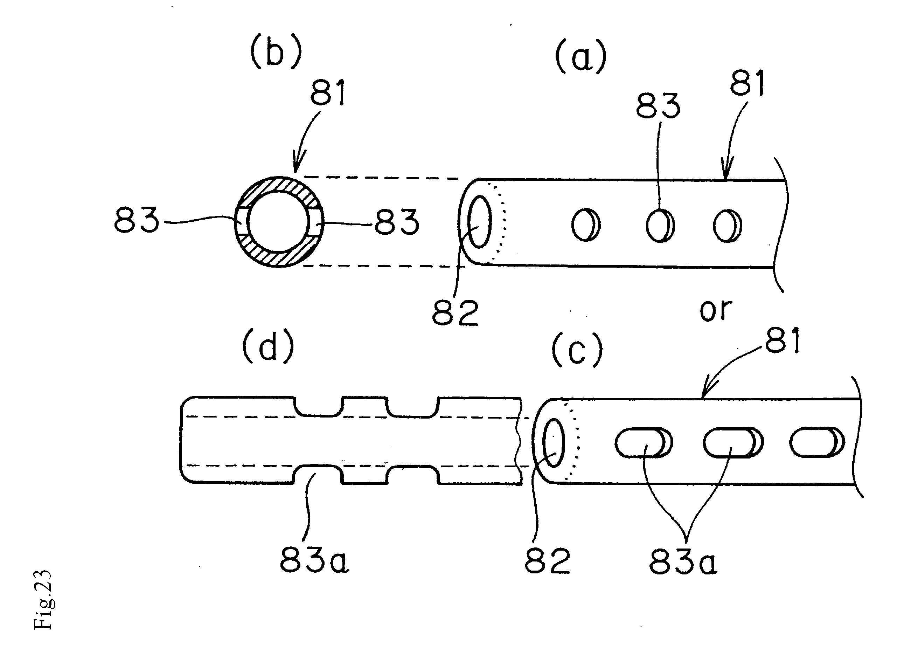

The catheter shown in Figure 22 is a single lumen type catheter which is, as shown in Figure 23a and Figure 23b, provided with an opening 82 of the front edge and further the lateral hole 83 passing through the neighborhood sidewall. A plurality of the lateral hole 83 is provided respectively at both sides in a row, and in this embodiment three each is provided. But it may be provided only at one side. One each, two each, or four each of them may be provided. Further, they may be not aligned in a row but may be in random arrangement. The lateral hole 83 is circular in Figure 23a, but the shape is not limited. For example as shown in Figure 23c, Figure 23d, it can be oval or elliptical shape lateral hole 83. Further, it can be the other shape as rectangular.

As shown in Figure 22, the catheter 81 is connected to the one end 67a of the straight extending tube of the T-shaped three way turncock 67, and it is used by connecting the infusion solution bag 64 for introducing the physiological salt solution to the upward tube 67b. The other end of the straight extending tube 67c is used for passing through the guide wire for guiding the catheter 81 into the bone, and for inserting the guide wire 81 for the bone marrow stirring, and furthermore for connecting the injection syringe to aspirate the mixed liquid of the physiological salt solution and the bone marrow. In other words, the lumen of the catheter 81 fills three roles; (1) a lumen to inject the physiological solution; (2) a lumen to insert into the stirring wire (or the guide wire to guide); (3) a lumen to harvest the mixed liquid of the physiological solution and the bone marrow.

The lever 67d of the three way turncock 67 is used for switching the operation between the condition in which the one end 67a and the other end 67c of the straight extending tube are communicating and the condition in which the one end 67a and the upward tube 67b are communicating.

The catheter 81 provided with the lateral hole 83 as described above can aspirates not only by the front opening 82 but also by the lateral hole 83, in the case that it aspirates the mixed liquid of the physiological salt solution and the bone marrow. Thereby it can harvest from the wide region around the neighborhood of the front edge of the catheter 81. Furthermore, the aspiration may be employed by moving the catheter 81 along the medullary cavity which enables efficient harvesting of the bone marrow in the medullary cavity. In addition, when the physiological salt solution is injected to the medullary cavity, it can inject widely around from the front edge, and can efficiently mix the bone marrow and the physiological salt solution.

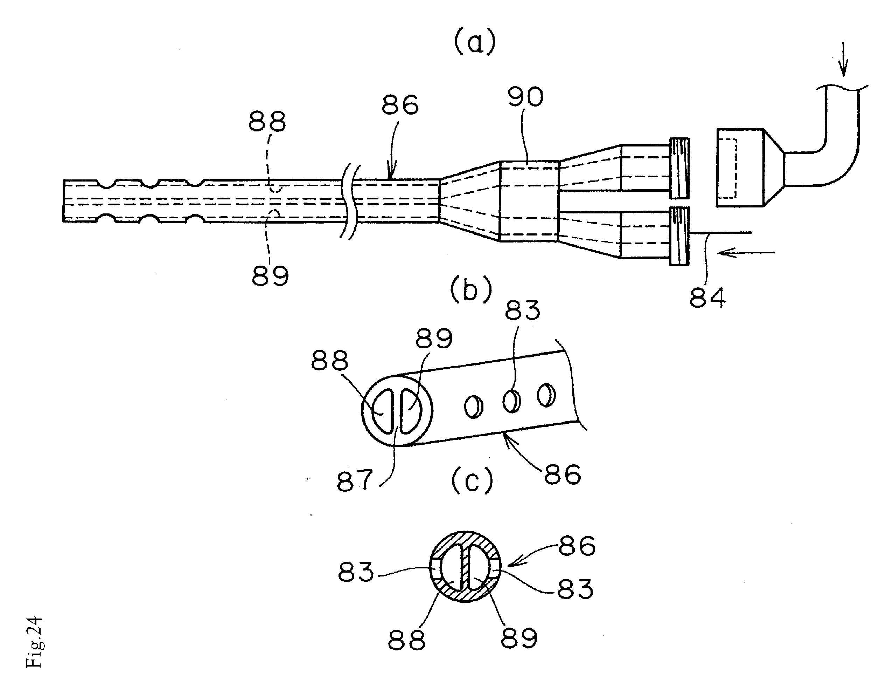

Figure 24 shows the embodiment of the double lumen type catheter 86. The catheter 86 is divided into two lumens 88, 89 by a partition 87 along the lengthwise, as shown in Figure 24b and Figure 24c. And in the sidewall in the vicinity of the front edge, the lateral hole 83 same as that of Figure 22 is formed. The lateral hole 83 can be provided on the bilateral sidewall sandwiching the partition 87, and it can also be provided on the one of the sidewall. Further, as shown in Figure 23c, Figure 23d, it can also be oval or elliptic, or rectangular.

This double lumen type catheter 86 is, for example as shown in Figure 24a, connected to a branch connector 90, and can stir the physiological salt solution and the bone marrow simultaneously while injecting the physiological salt solution by the upper lumen 88 of Figure 24a. The stirring wire 84 for stirring the bone marrow being passed through the lower lumen 89, and after the wire is taken out, an injection syringe for aspiration is connected enabling to aspirate the stirred mixed liquid. Further, after injecting the physiological salt water from the upper lumen 88, the stirring wire is inserted for stirring inside of the medullary cavity, and the mixed liquid can be aspirated from the lower lumen 89.

Both the above described single lumen type catheter 81 and the double lumen type catheter 86 may be used. The catheter 86 of Figure 24a is a double lumen provided with two lumens 88, 89, but it is possible to be a multi-lumen provided with more than three lumens. In this case, the operation of injecting the physiological salt solution, the operation of stirring inside of the medullary cavity, and the operation of aspirating the mixed liquid from the medullary cavity can be carried out simultaneously. And when it is carried out alternately, since there is no need of reconnecting the infusion solution bag, the wire, the injection syringe for aspiration or a cylinder and a vacuum pump, efficient harvesting of the bone marrow is possible.

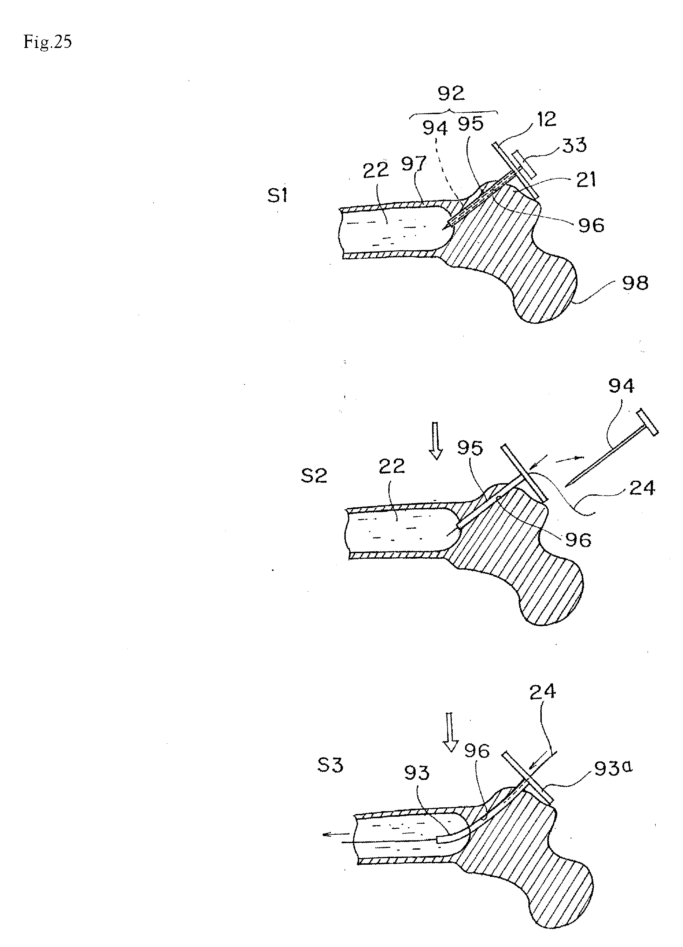

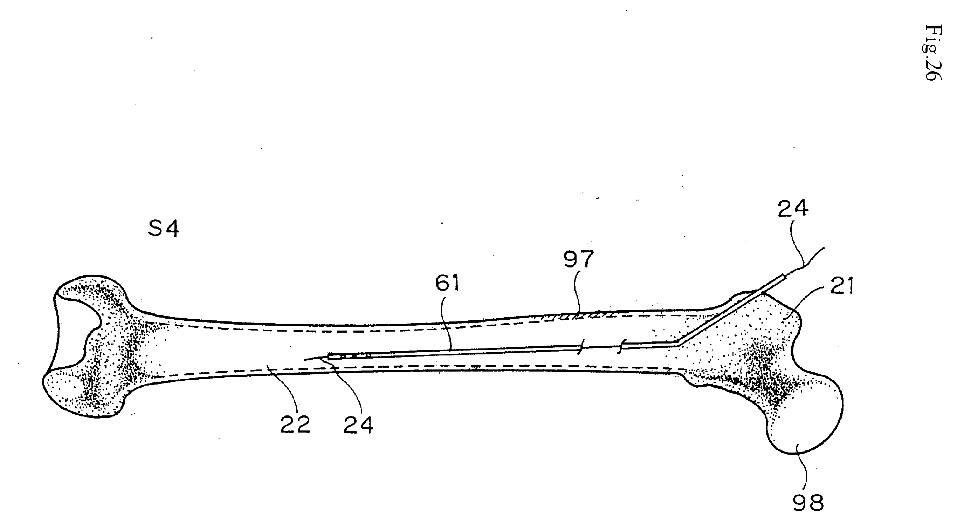

Next, referencing Figure 25 and Figure 26, the method of harvesting the bone marrow of this invention, particularly the other embodiment leading up to the insertion of the catheter is described. In the puncturing process of Figure 3, a bended hole is formed using the bended puncturing needle but in the case of Figure 25, the almost straight puncturing needle 92 and the pipe-shaped needle like device 93 are combined to be used.

More specifically, in the first step S1 of Figure 15, the puncturing needle 92 having the sheathed core structure composed of the straight inner needle 94, and the straight outer needle 95 where the inner needle is inserted extractably and rotatably, is used to form a straight hole 90 is in the greater trochanter 21. The hole 96 is formed in an inclined direction toward the medullary cavity 22 side. The inner needle 94 and the outer needle 95 are about the same as the inner needle 31 and the outer needle 32 of the puncturing needle 30 of Figure 6, but it is not bended and presents straight shape. Those are also the same as above that the front edge of the outer needle 95 or the inner needle 94 is shaped into cone, that the cutting blade is provided, that the grip 12 is provided on the outer needle 95, and that the knob 33 is provided on the inner needle 94. Because it has straight shape, the inner needle is not required to be flexible. Further, the inner needle 94 is not required to be hollow and may be solid. But it can be a hollow tube if necessary. Other than the puncturing needle having the sheathed core structure, the puncturing needle having no sheath same as Figure 1 can be used. In addition, the reference numeral 97 of Figure 15 is a cortical bone, and the reference numeral 98 is a caput.

In the second step S2 of Figure 25, the inner needle 94 is taken out and the guide wire 24 is inserted to the outer needle 95. The guide wire 24 may be the same as the case of Figure 4. In the case that the outer needle 95 is straight and the formed hole 96 is inclined, depending on the angle between the outer needle 95 and the medullary cavity, the guide wire 24 cannot be inserted into the depth of the medullary cavity 22.

In this case, the outer needle 95 is taken out leaving the guide wire 24, and the hollow pipe needle like device 93 is inserted (the third step S3 of Figure 25). The needle like device 93 is about the same as the exploring tube 60 of Figure 16, and it can also enlarge the hole 96 formed in the bone tissues. But, the whole or front edge of the needlelike device 93 is bended, and the inner cavity is opened at the front edge. Additionally, the opening may be opened laterally. In this case, it is preferable to be opened inside of the bend. The needle like device 93 is provided with flexibility and elasticity so as to pass the bended needle like device 93 through the straight hole 96. The needle like device 93 can be composed of metal or synthetic resin similarly to the exploring tube 60. In the end part of the needle like device 93, a grip 93a is attached.

When the needle like device 93 is passed through the hole 96 and the front edge enter the medullary cavity 22, the front edge of the needle like device 93 returns to its bended condition by elasticity. Thereby the direction of the guide wire 24 is pointed to the depth of the medullary cavity 22. Therefore, by advancing the guide wire 24, the front edge can be guided to the depth of the medullary cavity 22. These operations are carried out under the monitoring by roentgen rays.

Once the guide wire 24 is inserted into the depth of the medullary cavity 22, as shown in Figure 26, the needle like device 93 is taken out leaving the guide wire 24 in its condition, and the catheter 61 is inserted along the guide wire 24. Thereby the front edge of the catheter 61 is guided to the depth of the medullary cavity 22 (the forth step S4 of Figure 26). After that the physiological salt solution is injected into the bone marrow in the medullary cavity 22, and the bone marrow is aspirated to harvest while being stirred and mixed, in the same way as Figure 18 etc.

Since the method for harvesting the bone marrow using the above straight puncturing needle 92 and the bended needle like device has the straight puncturing needle 92, it is relatively easy to form a necessary hole 96 in a cortical bone which needs to apply a force to puncture. Further, since the needle like device 93 resembles the exploring tube as described above, the one needle can be used both as the exploring tube and the needle like device. As for the needle like device, not bended one provided with the same lateral opening as Figure 5a etc. can be employed. In this case, the guide wire is once taken out, and after the needle like device is inserted, the guide wire is inserted again into the inside cavity of the needle like device, and the front edge of guide wire from the lateral opening. Thereby, the direction of the guide wire can be pointed to the medullary cavity.

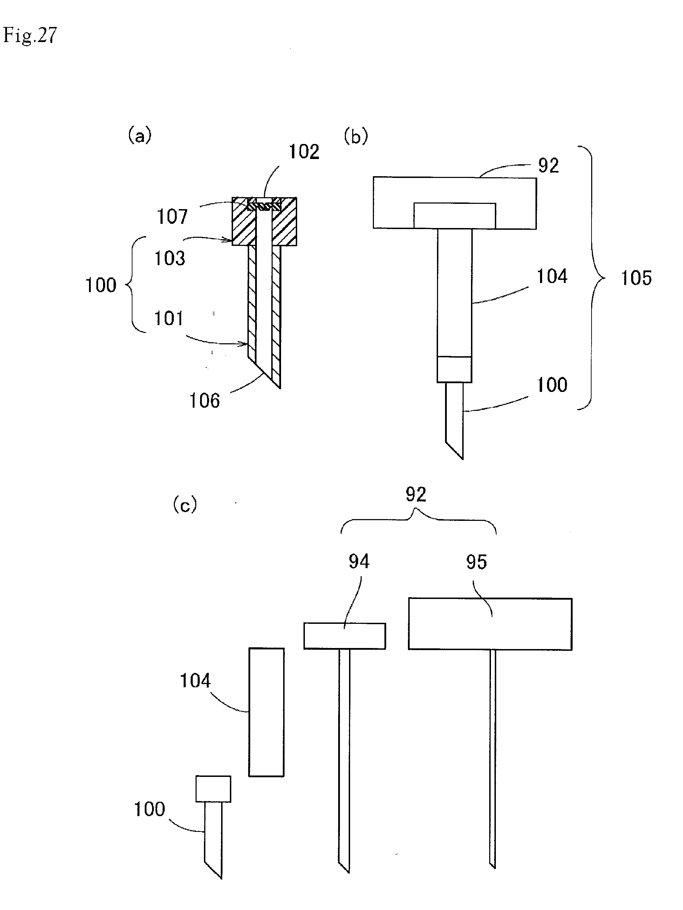

The punctured hole securing member which can be used in this invention of Figure 25 and Figure 26 is described.
The punctured hole securing member 100 shown in Figure 27a comprises a punctured hole securing needle 101 having a cavity and a flange part 103 having a communication hole 102 communicating with the cavity of the punctured hole securing needle. Further, an auxiliary attachment 104 having a cavity can be attached to the upper edge of the flange part 103 of the punctured hole securing member. And to the punctured hole securing member 100 to which the auxiliary attachment 104 is attached, the puncturing needle 92 is inserted and used as a bone marrow harvesting system 105 (see Figure 27a, 27b).

The puncturing hole securing needle 101 is provided with a cutting part 106 of which the lower end is sharp cone. This cutting part 106 presents a cut-shape cut at a slant at its lower end. Such provision enables to puncture the cortical bone while rotating the punctured hole securing needle 101. But the cutting part 106 may be multi-pyramid. Further, the cutting part 106 has the same shape as the cutting part 14 of the lower end of the puncturing needle 92, and it is so composed that when the puncturing needle 92 is attached to the auxiliary attachment 104, the plane of the cutting part 104 and the cutting part 106 is aligned. The punctured needle system 105 may be used in one body as a cone.

It is configured like this, but the punctured hole securing member 100 can be fixed by carrying out the following two process alternately; fixing the puncturing point by the cutting part 106 of the punctured hole securing needle and dugging the puncturing needle 92 into the cortical bone to some degrees by rotating the puncturing needle 92 in the punctured hole securing member 100; gugging the punctured hole securing needle 101 into the cortical bone by rotating the puncturing needle 92 while the puncturing point being fixed by the punctured hole securing needle 101. In this case, since the puncturing point is supported at two positions at the cutting part of the punctured hole securing needle 101 and the puncturing needle 92, the punctured hole securing member is securely fixed. Further, a return check valve 107 for permitting the flow from the flange part 103 to the punctured hole securing member 101 and stopping the adverse current is provided in the communicating hole 102 of the flange part. Thereby the flowing out of the periphery blood and the bone marrow liquid and infections are prevented.

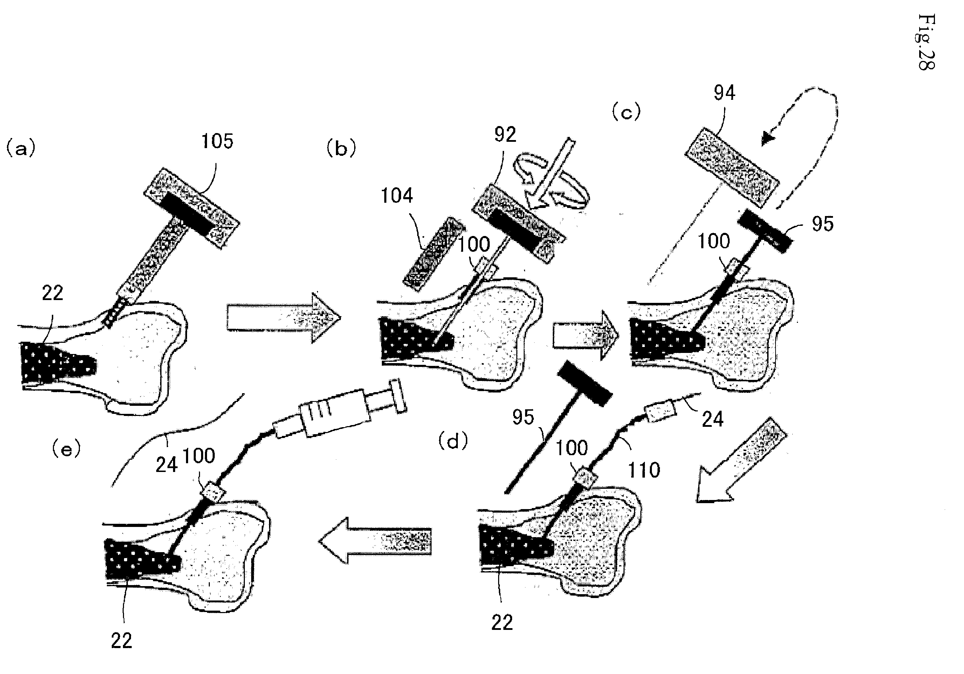

In the case of harvesting the bone marrow using the punctured hole securing needle 100, at first, the auxiliary attachment 104 is attached to the punctured needle securing member 100, the puncturing needle system 105 to which the puncturing needle 92 is inserted, is stuck to the greater trochanter of the bone (see Figure 28a). And then, when the front edge of the punctured hole securing needle 101 is dug into the cortical bone and fixed securely, the puncturing needle 92 is taken out, and the auxiliary attachment 104 is removed from the punctured hole securing member 100. Thereby, the punctured hole securing member 100 is fixed to the cortical bone. To the punctured hole securing member 100, the puncturing needle 92 is inserted again from the communicating hole 102 of the flange part, and puncturing is carried out until the front edge of the puncturing needle 92 reaches inside of the medullary cavity (see Figure 28b)

After puncturing by the puncturing needle 92, the inner needle 94 of the puncturing needle 92 is taken out and the guide wire 24 is inserted (see figure 28c). At this moment, there is some amount of angle between the direction of the guide wire 24 and the axis of the medullary cavity 22, but the front edge of the guide wire 24 advances along the sidewall of the medullary cavity 22 and reaches the depth of the medullary cavity 22. When the guide wire is difficult to enter due to the large puncturing angle, the use of the needle like device 93 having a cavity used in figure 25 allows easy insertion of the guide wire 24 to the depth of the medullary cavity 22. After the guide wire 24 is inserted, the outer needle 95 of the puncturing needle 92 is taken out, and the catheter 110 is inserted along the guide wire 24 (see Figure 28d). After that the bone marrow in the medullary cavity 22 is aspirated (see Figure 28e)

In this manner, since the punctured hole securing member 100 becomes an anchor for sticking the puncturing needle 92 to a bone after it is fixed to a cortical bone, it can serve to make the puncturing work safe preventing the puncturing needle from jumping laterally. By fixing the punctured hole securing member 100 to a bone at a slant, the puncturing needle 92 is easily stuck to the bone at the slant. Thereby, the guide wires and the catheters can be inserted to the depth of the medullary cavity without use of other devices, which is preferable.

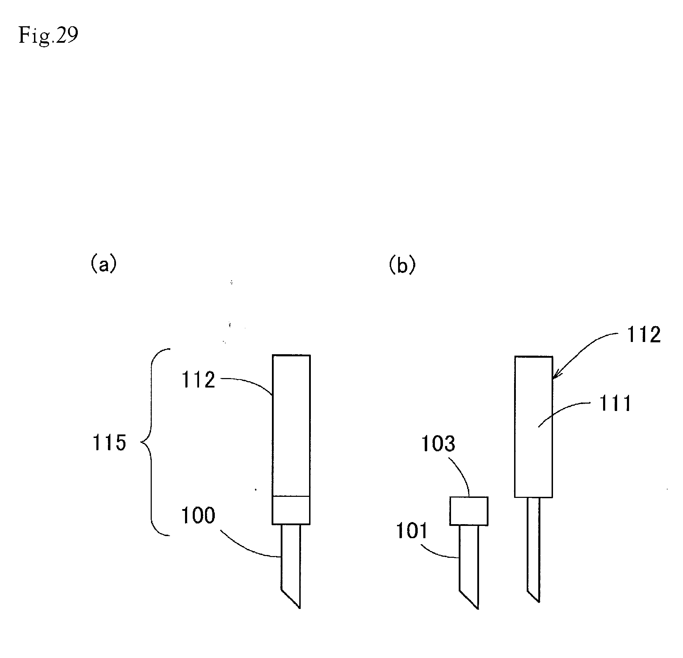

Further, the punctured hole securing member 100 may be fixed to the cortical bone by using the punctured hole securing needle system 115 in which an inner needle 112 having a grip 111 at its upper end is inserted (see Figure 29). This is achieved by sticking the punctured hole securing system 115 to the bone and extracting the inner needle 112. After fixing the punctured hole securing member 100, the puncturing needle 92 is inserted from the communication hole 102 of the flange part to reach the medullary cavity 22. After that, the guide wire and the catheter are inserted similarly like the bone marrow puncturing needle system of Figure 27a. In this case also, the bone marrow can be aspirated safely and securely similarly as the case the bone marrow puncturing system 105 is used.

Further, in the case that the punctured hole securing needle system 115 is used the punctured hole securing member 100 may be fixed with less puncturing resistance compared to the case in which the punctured hole securing member 100 is used alone. Because the punctured hole securing member 100 is fixed (punctured) to the cortical bone along with rotating the inner needle 112.. In this case also, there are two tooth points which are of the punctured hole securing member 100 and of its inner needle 112, the punctured hole securing system 115 is safely stuck without moving. But the inner needle 112 may be made not rotatable in the punctured hole securing member 100. In this case, similarly to the puncturing hole securing needle system 115, the punctured hole securing member 100 is fixed by being pushed while twisted.
The punctured hole securing member 100 may be formed in bended shape, and can be used for the puncturing needle 10 shown in Figure 1. Thereby, similarly, shifting or slipping laterally of the puncturing point resulting in to injure the internal organs can be prevented.

The above described bone marrow harvesting operation must be carried out wholly under the aseptic cleanness. Since the insertion of a catheter to the bone marrow may cause the infection incurring danger of osteomyelitis, sufficient caution is required to prevent the infections. The configuration of the medical apparatus of this invention and the practical embodiments of these usages are described bellow.

### Embodiment 1

The outer needle of the outer diameter 2.2mm, inner diameter 2mm, and length 7cm and the inner solid needle of the outer diameter 1.9mm, length 7.2mm were combined into a puncturing needle made of stainless steel having a curve of radius 5cm. Its front edge was formed into three-plane cone. The outer needle and the inner needle were made to be in an interlocked condition at its near end which was the opposite side of the front edge. It was so constructed that turning a bit clockwise made fastening and turning a bit anti-clockwise made loosening. This was made to be the bone marrow needle for the test use.

The exploring tube of the outer diameter 2.2mm, inner diameter 1.5mm, and length 12cm was made of polyethylene. This exploring tube had bendability, and further, the sufficient strength for puncturing spongy bones.

A catheter of the outer diameter 2.2mm, inner diameter 2mm, and length 25 cm made of polyethylene was prepared. A bend of about 120 degree was made respectively at the position 1cm, 2cm, and 3cm apart from its front edge by thermal processing. Further, one lateral opening of the diameter of 2mm was made at the position 0.7cm apart from its front edge. At the near end, a three-way turncock is attached, and at its end, adverse current check valve used for the vessel catheter operation was attached.

The same size catheter made of polyethylene was prepared. To this, a three-way turncock was attached at the near side, and its end was received by an adverse current check valve used for the vessel catheter operation, but any bend or any lateral opening was not attached. On the other hand, a guide wire of the outer diameter 0.4mm, length 40cm was prepared. For this, a guide wire for the angiography was used of which the one end has flexibility and the other end has rigidity.

A wire composed of multi-filaments of a piano wire of the diameter 0.5mm, length 30cm was prepared. A bend of 120 degree was made 0.5cm, 1.5cm, and 2.5cm apart form its front edge respectively. It is made to be a stirring rod.

A male dog of the body weight 20 kg was subjected to the general anesthesia, and was laid at a right lateral recumbent posture. The greater trochanter region of the femur of left lumbar was observed by the palpation, and the above prepared puncturing needle was stuck under cleanness. A weak force was applied to cut the skin at the start and to puncture to the greater trochanter, but after that, the needle was pushed forward just like the cone was being pushed while being turned, thereby the font edge of the puncturing wire punctured the cortical bone and the puncturing needle was stuck into the femur.

Thereupon, the front edge of the needle was pushed forward to bend along the curve of the needle in the direction of the long axis, and when the front edge was punctured about 3cm toward the medullary cavity in the bone, the inner needle was taken out and the prepared guide wire was inserted. At this moment there was a resistance, but it was kept advancing strongly, and at the position about 1cm forward, the resistance diminished allowing further advance of about 20cm. Here, the position of the guide wire was observed by seeing through using roentgen rays, and it was verified that the guide wire was in the medullary cavity of the femur, and that its front edge reached near the articulation genus.

Then, the puncturing needle was taken out, and in place of it the exploring tube was inserted. There was a resistance a little, but it was inserted about 6cm and then taken out. Further, to the prepared catheter provided with the bend and the lateral opening, an infusion solution bag charged with a physiological salt solution was connected in the condition capable of the intravenous drip infusion via a three-way turncock, and the catheter was inserted along the guide wire so as to cover it, and at the position of about 20 cm insertion, the guide wire was taken out.

And then, by the similar operation, the puncturing needle was stuck to the spongy bone region after breaking the cortical bone in the vicinity of the greater trochanter. After taking out the inner needle, the guide wire was inserted to the medullary cavity via the outer needle. After verifying this by seeing through by roentgen rays, the outer needle was taken out, and in place of this, the exploring tube having bendability substituting the outer needle was inserted while being turned so as to cover the guide wire. This operation was carried out easily. Then the exploring tube was taken out, and in place of it, to the catheter without the lateral opening and the bend, an infusion solution bag charged with a physiological salt solution was connected in the condition capable of the intravenous drip infusion via a three-way turncock, and the catheter was inserted, and at the position of about 20 cm insertion, the guide wire was taken out.

Then, the stirring rod prepared in the secondly inserted catheter was inserted about 24cm. At this point, the position of the stirring rod and the catheter was verified seeing through by roentgen rays. The stirring rod was verified to be in the medullary cavity about 5cm out of the front edge of the catheter. By rotating the stirring rod at the near side, the front edge was verified to be rotated accordingly.

Next, from the three way turncock of the catheter with lateral hole, aspirating of suspension of the bone marrow by the injection syringe of 20 ml, along with gradually injecting 16 ml of the physiological salt water, and stirring the marrow cavity by stirring rod was employed. As a result, the suspension of the bone marrow tinged with yellow was aspirated amounting 27ml. Further, the suspension was subjected to the centrifugal separation of two minutes, 100 rotations per minute, thereby the physiological salt solution was separated.

The harvested suspension of the bone marrow was observed by an optical microscope, and mega-karyocytes and cells inherent to the bone marrow such as erythroblast were found abundantly. Thereby it was proved that a large amount of the bone marrow was contained in the suspension. The operation of the embodiment 1 was proved to be capable of harvesting easily a large amount of the bone marrow from femurs.
Further, the postoperative observation of the operated dog was continued. No appearance of being pained in the left leg, no appearance of being conscious was observed, and further, any fever and anorexia was not observed. Therefore, affection of this treatment to the whole body or a part of the body is estimated to be extremely small.

### Embodiment 2

Using another dog of the body weight 20kg. the bone marrow harvesting was carried out under the general anesthesia. This time also, the puncturing needle, exploring tube, catheter, guide wire etc. are prepared. But, as the catheter, a double lumen catheter was prepared, which comprised, despite of the outer diameter of 2.2mm similar to the embodiment 1, a circular main tube of the inner diameter 1.4mm and a side tube composed of the thin gap of the thickness 0.4mm provided on its side.

A three-way turncock was connected to the both tubes respectively at its near side, to which an infusion solution bag charged with a physiological salt solution is connected via the each connecting tube in the condition capable of the drip infusion, and the catheter was charged with the physiological salt solution. In a part of the three-way turncocks, an adverse current check valve used for the vessel catheter operation was attached. This catheter has a lateral hole 0.7mm apart from the front edge and communicates with the main tube of the diameter 1.4mm. A 120 degree bend was attached by thermal processing at the position 1cm, 2.5mm, and 4mm apart from the front edge respectively.

The animal was laid at a right lateral recumbent posture. The greater trochanter region of the femur of left lumbar was observed by the palpation, and the prepared puncturing needle was stuck under cleanness. A weak force was applied to cut the skin at the start and to puncture to the greater trochanter, but no strong force was necessary. After that, the needle was pushed forward while being turned just like using a drill, a small resistance appeared proving that the front edge of the puncturing needle entered the bone.

Then, the inner needle was taken out, and in place of it, the guide wire was inserted and pushed with a force, the resistance disappeared at the insertion of about 4cm allowing further insertion of 20cm. And then, the outer needle was taken out and the exploring tube was inserted and pushed while rotated to the position of about 4cm. Thereupon, the operation was stopped and the exploring tube was removed.

The prepared catheter was inserted along the guide wire. A small resistance appeared at the position where the front edge entered about 4cm, but by applying a weak force, the resistance diminished allowing further insertion. At the point of 20cm insertion, the insertion was stopped and the exploring tube was taken out.

Then, the push and pull operation of the catheter while rotating was repeated many times, dropping the physiological salt solution from the side tube of the catheter gradually, and carrying out this operation, the aspiration by the injection syringe was done to harvest 31ml of liquid in which the bone marrow was mixed. The amount of injected physiological salt solution was 20ml at this point.

The harvested liquid was observed by an optical microscope and the cells inherent to the bone marrow were found proving that this was the suspension of the bone marrow. In this liquid, many erythrocytes were found, but it was visually not bright red as that of blood, and it was muddy liquid mixed with sludgy pieces of tissue. By this treatment, it became apparent that even eyeless puncturing to the greater trochanter region without seeing through by roentgen rays enables to insert the catheter to the medullary cavity certainly, and that secure and a large amount harvesting of the bone marrow was possible. Further, according to the postoperative observation, no bad condition was observed similarly to the embodiment 1, proving the safety of this treatment.

### Embodiment 3

The bone marrow puncturing to an animal was carried out to prove the effect using the puncturing needle having the similar curve without inner needle. The puncturing needle used was only the outer needle of the outer diameter 2.2mm, inner diameter 2mm, and length 7cm, having no inner needle. The front edge was made to be three-plane cone. A lateral hole of the width 0.6mm, length 2mm was provided inside of the curve of the needle. The near side of the needle was so constructed as to enable the attachment of an infusion fringe. The guide wire, exploring tube to be used at the same time was the same as that used in the embodiment 2, and the catheter of double lumen having the curved structure in its front edge was used.

At first, the animal was laid at a right lateral recumbent posture same as the method of the embodiment 2. The greater trochanter region of the femur of left lumbar was observed by the palpation, and the prepared puncturing needle was stuck under cleanness. A weak force was applied to puncture the skin and the bone surface, but no strong force was necessary. After that, the needle was pushed forward while being turned just like using a drill, a small resistance appeared proving that the needle point reached the spongy bone after passing through the cortical bone. Thereupon, the needlepoint was pushed about 4cm toward the diaphysis and stopped, and the guide wire was pushed in the puncturing needle. A small resistance appeared there, but by applying a bit of force to push, the resistance diminished at about 5cm further advance, allowing further easy advance of about 20cm.

Then, the puncturing needle was taken out, and the exploring tube was inserted and pushed forward while rotated to the position of 5cm. There, the operation was stopped, and the guide wire was taken out. The exploring tube was attached to the injection syringe and a small negative pressure was applied, resultantly the bone marrow like tissue was harvested. With that, the guide wire was again inserted and the exploring tube was taken out.

Then, the prepared catheter was inserted along the guide wire. There was a small resistance, but by applying a bit of force to push, the resistance diminished allowing further advance. At the point of 20cm insertion, the insertion was stopped and the guide wire was taken out.

Then, same as the embodiment 2, the catheter was rotated in the medullary cavity and moved back and forth at the same time, simultaneously a negative pressure was applied while a physiologic salt solution was injected by the injection syringe, the liquid of suspended particulate of the bone marrow was easily harvested. There, the amount of the injected physiologic salt solution was 30ml, and the amount of harvested liquid of suspended particulate of the bone marrow was 39ml. The liquid of suspended particulate of the bone marrow was subjected to the centrifugal separation, and the pure bone marrow liquid was collected.

According to the observation of the animal treated, no symptom of side-effects was found. From this fact it became apparent that when the needle has a curve, even if it is the puncturing needle without the inner needle, bone chips are prevented to enter into the lateral groove, and that the harvesting a large amount of the bone marrow by inserting the catheter safely and securely from the side of the epiphysis is possible.

### Embodiment 4

On the assumption that bone chips enter into the lateral hole of the puncturing needle, experimentally a part of the spongy bone was taken and pushed in the lateral hole by a force. Being left as just described, the guide wire was inserted and pushed by a force, and the plugged bone chips were easily removed. Accordingly, it was proved that even if bone chips were clogged in the lateral hole of the puncturing needle, the guide wire could push them out. Therefore, it was found that even the inner needle was not provided, the bone chips were hard to enter into the lateral hole, and that even if they entered into the hole, the guide wire could easily push them out.

Regarding the position of the lateral hole of the puncturing needle, if it is located at any position, a small distance from the front edge makes it hard for the bone chips to enter into the hole, arising no problem. Further, it became apparent in the experiment that the position of the lateral hole was formed inside of the curve, even if the front edge of the puncturing needle was not perfectly directed to the bone marrow in the spongy bone, the front edge of the guide wire pushed into the puncturing needle was easy to be directed toward the medullary cavity.

## Claims

1. A method for harvesting a bone marrow, comprising a steps of forming a hole in a cortical bone, inserting a flexible catheter into a bone marrow cavity through the hole, and aspirating the bone marrow through the catheter.

2. A method for harvesting a bone marrow according to claim 1, further comprising a steps of injecting a dilute solution to the bone marrow cavity through the catheter, stirring the bone marrow and dilute solution, and aspirating the bone marrow as a mixed liquid.

3. A method for harvesting a bone marrow according to claim 1, further comprising a steps of passing a puncturing needle through from a epiphysis of a long tubular bone to a spongy bone or bone marrow cavity, inserting a guide wire into the bone marrow cavity through from a cavity of the puncturing needle, extracting the puncturing needle leaving the guide wire, inserting the flexible catheter with guidance of the guide wire, and aspirating the bone marrow through the catheter.

4. A method for harvesting a bone marrow according to claim 3, further comprising a steps of fixing or contacting a punctured hole securing member having a cavity to a cortical bone, before inserting the puncturing needle into the spongy bone or bone marrow cavity through the cavity of the punctured hole securing member.

5. A method for harvesting a bone marrow according to claim 3, further comprising a steps of inserting an exploring tube, confirming that the exploring tube has reach the bone marrow cavity by aspirating the exploring tube, before inserting the flexible catheter after extracting the puncturing needle.

6. A method for harvesting a bone marrow according to claim 3, further comprising a steps of inserting a needle like device having a cavity and bended front edge with a guidance of the guide wire, and further inserting the guide wire deeply into the bone marrow cavity using the needle like device.

7. A combined set of a medical apparatus for harvesting a bone marrow, comprising; a puncturing needle composed of an outer needle having a cavity provided with rigidity and an inner needle to be inserted into the outer needle; and a tube like flexible catheter inserted into the outer needle or a hole of a bone formed by the outer needle.

8. A combined set of a medical apparatus for harvesting a bone marrow, comprising; a puncturing needle composed of an outer needle having a cavity provided with rigidity and an inner needle to be inserted into the outer needle; a guide wire which can be inserted into the outer needle of the puncturing needle; and a needle like device having a cavity for inserting the guide wire, having a bended part with the cavity, and capable of being inserted into a hole formed by the puncturing needle.

9. A combined set of a medical apparatus for harvesting a bone marrow according to claim 8, further comprising; a fixing means for fixing to the cortical bone, and a punctured hole securing member having cavity.

10. A puncturing needle for harvesting a bone marrow, having a cavity provided axially for inserting a guide wire, wherein the puncturing needle may be provided with a bended or without a bended part.

11. A puncturing needle for harvesting a bone marrow according to claim 10,
wherein a front edge is formed into multi-pyramid cone and the cavity is opened at the vicinity of the front edge.

12. A puncturing needle for harvesting a bone marrow according to claim 10 or 11, wherein the cavity is opened at an inside of the bended puncturing needle.

13. A puncturing needle for harvesting a bone marrow, comprising; an outer needle having a cavity provided axially for inserting a guide wire with the cavity opened at a front edge where the outer needle may be provided with a bended or without a bended part; a flexible inner needle accommodated rotatably and extractably in the cavity of the outer needle,
wherein a front edge of the inner needle has a cone shape, and a base of the inner needle is protruded from the outer needle so as to be operatable when the inner needle is accommodated in the outer needle presenting a sheathed core structure as a whole.

14. A puncturing needle for harvesting a bone marrow according to claim 13, further comprising an interlock mechanism provided in a part of the outer needle, which allows a rotation or reciprocation of the inner needle and at least restrains an axial movement toward a back end.

15. A catheter for harvesting a bone marrow used together with any of the needle described in claim 10 to 14, having an inner hole for a guide wire to be inserted and has a flexibility to advance the periphery of the guide wire in bended condition.

16. A catheter for harvesting a bone marrow according to claim 15, having a coupling part to inter connect an aspirating means to be connected to the inner hole, a liquid injecting part, and switching means at least at a par of the near side.

17. A catheter for harvesting a bone marrow, having a lumen for injecting a dilute solution, and a lumen for aspirating a diluted bone marrow.

18. A catheter for harvesting a bone marrow according to claim 15, 16, or 17, having a stirring part provided in vicinity of the front edge.

19. A catheter for harvesting a bone marrow according to claim 15, 16, 17, or 18, having at least one lateral hole for aspirating the bone marrow provided in vicinity of the front edge.

20. A guide wire for harvesting a bone marrow and for inserting into the catheter described in any of claim 15 to 19, having a one end having flexibility and an another end having hardness or rigidity.

21. A guide wire for harvesting a bone marrow and for inserting into the catheter described in any of claim 15 to 19, having substantially the same rigidity along a whole length.

22. A guide wire for harvesting a bone marrow and for inserting into the catheter described in any of claim 15 to 19, having a stirring part in vicinity of the front edge.

23. A set of a medical apparatus for harvesting a bone marrow, comprising; a puncturing needle described in any of claim 10 to 14, a guide wire which can be inserted into the puncturing needle, a bendable catheter which lead a periphery of the guide wire.

24. A set of a medical apparatus for harvesting a bone marrow according to claim 23, further comprising an exploring tube having bendable front edge with hardness slidably attached to the periphery of the guide wire.

25. A set of a medical apparatus for harvesting a bone marrow according to claim 23 or 24, further comprising a fixing means fixing to the cortical bone, and a punctured hole securing member having a cavity.
